(19) Europäisches Patentamt
European Patent Office
Office européen des brevets

(11) Veröffentlichungsnummer: **0 318 894 B1**

(12) # EUROPÄISCHE PATENTSCHRIFT

(45) Veröffentlichungstag der Patentschrift: **16.02.94**

(51) Int. Cl.⁵: **C07F 17/00**, C07D 207/32, C08F 2/50, G03F 7/027

(21) Anmeldenummer: **88119799.0**

(22) Anmeldetag: **28.11.88**

(54) **Titanocene, deren Verwendung und N-substituierte Pyrrole.**

(30) Priorität: **01.12.87 CH 4683/87**

(43) Veröffentlichungstag der Anmeldung:
**07.06.89 Patentblatt 89/23**

(45) Bekanntmachung des Hinweises auf die Patenterteilung:
**16.02.94 Patentblatt 94/07**

(84) Benannte Vertragsstaaten:
**AT BE CH DE FR GB IT LI NL SE**

(56) Entgegenhaltungen:
**EP-A- 0 119 162** **EP-A- 0 122 223**
**EP-A- 0 186 626** **EP-A- 0 207 893**
**EP-A- 0 242 330** **EP-A- 0 269 573**
**DE-A- 3 715 181**

**Chemical Abstracts, Band 107, 1987, Seite 597, Zusammenfassung Nr. 23190d, Columbus, Ohio, US; M.N. CHANG et al.: "Syntheses and analgesic/antiinflammatory activities of novel 2-(5-aroylpyrrolyl)alkanoic acids" & EUR J. MED. CHEM.--CHIM. THER. 1986, 21 (5), 363-9**

(73) Patentinhaber: **CIBA-GEIGY AG**
**Klybeckstrasse 141**
**CH-4002 Basel(CH)**

(72) Erfinder: **Hüsler, Rinaldo, Dr.**
**Route du Confin 52**
**CH-1723 Marly(CH)**
Erfinder: **Klingert, Bernd, Dr.**
**Möndenweg 87**
**D-7854 Inzlingen(DE)**
Erfinder: **Rembold, Manfred, Dr.**
**Im Aeschfeld 21**
**CH-4147 Aesch(CH)**
Erfinder: **Steiner, Eginhard, Dr.**
**Obere Hofackerstrasse 3**
**CH-4414 Füllinsdorf(CH)**

(74) Vertreter: **Zumstein, Fritz, Dr. et al**
**Patentanwälte,**
**Dr. F. Zumstein,**
**Dipl.-Ing. F. Klingseisen,**
**Bräuhausstrasse 4**
**D-80331 München (DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services
(3.10/3.09/3.3.3)

## Beschreibung

Die vorliegende Erfindung betrifft Titanocene mit fluorierten und mit Pyrrol substituierten aromatischen Resten, eine photopolymerisierbare Zusammensetzung aus ethylenisch ungesättigten Verbindungen, die diese Titanocene als Photoinitiatoren enthalten, ein mit dieser Zusammensetzung beschichtetes Substrat, ein Verfahren zur Herstellung photographischer Reliefabbildungen unter Verwendung dieses beschichteten Substrates und mit Fluoraromaten substituierte Pyrrole.

Aus der EP-A-0 122 223 ist es bekannt, dass Titanocene mit Fluorarylliganden ausgezeichnete Photoinitiatoren sind. Die Fluorarylliganden dieser Titanocene können mit Heterocycloaliphaten substituiert sein. Die Substitution mit Heteroaryl ist nicht erwähnt.

Ein Gegenstand der Erfindung sind Titanocene der Formel I

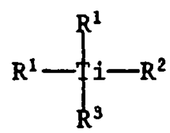

(I),

worin beide $R^1$ unabhängig voneinander gegebenenfalls ein- oder mehrfach durch $C_1$-$C_{18}$-Alkyl oder -Alkoxy, $C_2$-$C_{18}$-Alkenyl, $C_5$-$C_8$-Cycloalkyl, $C_6$-$C_{16}$-Aryl, $C_7$-$C_{16}$-Aralkyl, $SiR^4{}_3$, $GeR^4{}_3$, Cyano oder Halogen substituiertes Cyclopentadienyl$^{\ominus}$, Indenyl$^{\ominus}$, 4,5,6,7-Tetrahydroindenyl$^{\ominus}$ bedeuten oder beide $R^1$ zusammen für einen gegebenenfalls wie zuvor substituierten Rest der Formel II

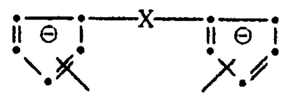

(II)

stehen, worin X $-(CH_2)_n-$ mit n = 1, 2 oder 3, gegebenenfalls durch Phenyl substituiertes Alkyliden mit 2 bis 12 C-Atomen, Cycloalkyliden mit 5 bis 7 Ringkohlenstoffatomen, $SiR^4{}_2$, $SiR^4{}_2$-O-$SiR^4{}_2$, $GeR^4{}_2$ oder $SnR^4{}_2$ ist, und $R^4$ $C_1$-$C_{12}$-Alkyl, $C_5$-$C_{12}$-Cycloalkyl, $C_6$-$C_{16}$-Aryl oder $C_7$-$C_{16}$-Aralkyl bedeutet,

$R^2$ einen 6-gliedrigen carbocyclischen oder 5- oder 6-gliedrigen heterocyclischen aromatischen Ring bedeutet, der in mindestens einer der beiden ortho-Stellungen zur Titankohlenstoffbindung mit Fluoratomen substituiert ist und wobei der aromatische Ring weitere Substituenten enthalten kann, $R^3$ unabhängig die Bedeutung von $R^2$ hat oder $R^2$ und $R^3$ zusammen einen Rest der Formel III bedeuten,

-Q-Y-Q- (III)

in dem Q für einen carbocyclischen aromatischen Ring steht, wobei die beiden Bindungen jeweils in Orthostellung zur Y-Gruppe stehen und die zweite Orthostellung zur Titankohlenstoffbindung jeweils durch ein Fluoratom substituiert ist und wobei Q weitere Substituenten enthalten kann, und Y $CH_2$, Alkyliden mit 2 bis 12 C-Atomen, Cycloalkyliden mit 5 bis 7 Ringkohlenstoffatomen, $NR^4$, O, S, SO, $SO_2$, CO, $SiR^4{}_2$, $GeR^4{}_2$ oder $SnR^4{}_2$ bedeutet und $R^4$ die zuvor angegebene Bedeutung hat, wobei die Titanocene dadurch gekennzeichnet sind, dass $R^2$ und $R^3$ durch einen Rest der Formel IV substituiert sind,

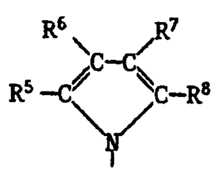

(IV)

worin $R^5$, $R^6$, $R^7$ und $R^8$ unabhängig voneinander ein Wasserstoffatom, unsubstituiertes oder mit $C_2$-$C_8$-Dialkylamino oder -aminomethyl, Bis[2-($C_1$-$C_4$-alkoxy)ethyl]amino oder -aminomethyl, Morpholino, Morpholinylmethyl, Piperidino, Piperidinylmethyl, N-Methylpiperazino, N-Methylpiperazinylmethyl, Pyrrolidino, Pyrrolidinylmethyl, quaternärem $C_3$-$C_{10}$-Trialkylammonium oder -ammoniummethyl, $C_1$-$C_{12}$-Alkoxy,

$$-(OCH_2CH_2)_l-O-C_1-C_{16}\text{-Alkyl},$$

worin 1 eine Zahl von 1 bis 20 ist, 1,3-Dioxolan-2-yl-, 4-Methyl-1,3-dioxolan-2-yl, $-OCH_2CH_2O-$, $C_2$-$C_{12}$-Alkoxycarbonyl, $C_2$-$C_{12}$-Alkanoyloxy, $C_2$-$C_{12}$-Alkanoyl, $C_1$-$C_{12}$-Alkylthio, Halogen, Cyano oder $SiR^4{}_3$, worin $R^4$ die zuvor angegebene Bedeutung hat, substituiertes lineares oder verzweigtes $C_1$-$C_{18}$-Alkyl, $C_2$-$C_5$-Alkenyl, $C_7$-$C_9$-Aralkyl oder -Alkaryl, $C_8$-$C_{10}$-Alkaralkyl, $C_6$-$C_{10}$-Aryl, 2-Furyl, $C_5$-$C_8$-Cycloalkyl, $C_5$-$C_8$-Cycloalkenyl, $C_2$-$C_{12}$-Alkanoyl oder $C_2$-$C_{12}$-Alkoxycarbonyl bedeuten; oder -CHO, $-SiR^4{}_3$ oder $-GeR^4{}_3$ darstellen, worin $R^4$ die zuvor angegebene Bedeutung hat; oder $R^5$ und $R^6$ und/oder $R^7$ und $R^8$ oder $R^6$ und $R^7$ jeweils zusammen $-(CH_2)_3-$, $-(CH_2)_4-$, -CH = CH-CH = CH-, $-CH=CH-C(R^{11})=CH-$, $-CH_2OCH_2-$oder $-CH_2N(C_1\text{-}C_4\text{-Alkyl})CH_2-$ sind, worin $R^{11}$ Hydroxyl, $C_1$-$C_4$-Alkoxy oder $C_2$-$C_4$-Alkanoyloxy bedeutet.

Bei den Gruppen $R^1$ handelt es sich bevorzugt um gleiche Reste. Als Substituenten kommen für $R^1$ in Frage: lineares oder verzweigtes Alkyl oder Alkoxy mit 1 bis 18, besonders 1 bis 12 und insbesondere 1 bis 6 C-Atomen, und Alkenyl mit 2 bis 18, besonders 2 bis 12, und insbesondere 2 bis 6 C-Atomen, wie z.B. Methyl, Ethyl, Propyl, Isopropyl, n-Butyl, tert.-Butyl, Pentyl, Hexyl, Octyl, Decyl, Dodecyl, Tetradecyl, Hexadecyl, Octadecyl und entsprechende Alkenyl-und Alkoxygruppen; Cycloalkyl mit 5 bis 8 Ringkohlenstoffatomen wie z.B. Cyclopentyl, Cyclohexyl, Cycloheptyl, Methylcyclopentyl und Methylcyclohexyl; Aryl mit 6 bis 16 C-Atomen und Aralkyl mit 7 bis 16 C-Atomen wie z.B. Phenyl, Naphthyl, Benzyl und Phenylethyl; Cyano und Halogen, besonders F, Cl und Br; $SiR^4{}_3$ oder $GeR^4{}_3$ worin $R^4$ bevorzugt $C_1$-$C_8$-Alkyl, Cyclohexyl, Phenyl oder Benzyl ist. Beispiele für $R^4$ in der Bedeutung von Alkyl sind Methyl, Ethyl, n- und i-Propyl, n-, i-und t-Butyl, Pentyl, Hexyl, Heptyl und Octyl.

Die Reste $R^1$ können bis zu 5, besonders aber bis zu 3 Substituenten enthalten. Insbesondere sind beide $R^1$ Cyclopentadienyl$^{\ominus}$- oder Methylcyclopentadienyl$^{\ominus}$-Reste.

X in Formel II enthält in seiner Bedeutung als Alkyliden bevorzugt 2 bis 6 C-Atome. Beispiele für Alkyliden, das gegebenenfalls durch Phenyl substituiert sein kann, und Cycloalkyliden sind Ethyliden, Propyliden, Butyliden, Hexyliden, Phenylmethylen, Diphenylmethylen, Cyclopentyliden und Cyclohexyliden. $R^4$ in der Gruppe X in seiner Bedeutung als Alkyl enthält bevorzugt 1 bis 6 C-Atome, und ist z.B. Methyl, Ethyl, Propyl, Butyl oder Hexyl, und ist in seiner Bedeutung als Cycloalkyl bevorzugt Cyclopentyl oder Cyclohexyl, in seiner Bedeutung als Aryl bevorzugt Phenyl und in seiner Bedeutung als Aralkyl bevorzugt Benzyl. X in der Bedeutung von

$$-(CH_2)_n-$$

ist bevorzugt Methylen.

Bei $R^2$ in seiner Bedeutung als 6-gliedriger carbocyclischer aromatischer und Fluor-substituierter Ring handelr es sich um Phenyl. $R^2$ als heterocyclischer aromatischer und 5-gliedriger Rest enthält bevorzugt ein Heteroatom und als 6-gliedriger Ring bevorzugt 1 oder 2 Heteroatome. Bevorzugt sind beide Orthostellungen mit Fluor substituiert. Beispiele sind 4,6-Difluorinden-5-yl, 5,7-Difluorindan-6-yl, 2,4-Difluorfluoren-3-yl, 1,3-Difluornaphth-2-yl, 2,6-Difluorphen-1-yl, 2,4-Difluorpyrr-3-yl,2,4-Difluorfur-3-yl, 2,4-Difluorthien-3-yl, 2,4-Difluorpyrrid-3-yl,4,6-Difluorpyrimidin-5-yl, 3,5-Difluorpyridazin-4-yl.

$R^2$ und $R^3$ zusammen als Rest der Formel III können z.B.

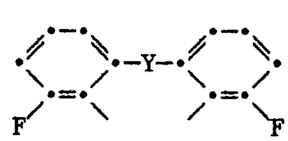

sein. Y in Formel III und in obiger Formel ist bevorzugt Methylen, Ethyliden, Propyliden, S oder O. $R^3$ hat bevorzugt die gleiche Bedeutung wie $R^2$.

In einer bevorzugten Ausführungsform ist in Formel I $R^2$ substituiertes 2,6-Difluorphen-1-yl oder $R^2$ und $R^3$ zusammen ein substituierter Rest der Formel

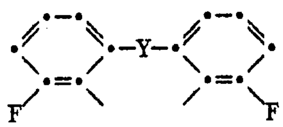,

worin Y die zuvor angegebene Bedeutung hat, die mit einem Rest der Formel IV substituiert sind und weitere Substituenten enthalten können. Insbesondere ist $R^2$ 2,6-Difluorphen-1-yl, das 1 bis 3 weitere Substituenten enthält, wovon mindestens einer ein Rest der Formel IV ist.

Eine bevorzugte Ausführungsform ist dadurch gekennzeichnet, dass $R^2$ und $R^3$ für 2,6-Difluorphen-1-yl stehen, an das ein Rest der Formel IV gebunden ist, und das weitere 1 oder 2 gleiche oder verschiedene Substituenten enthalten kann.

Eine bevorzugte Gruppe von Metallocenen der Formel I sind solche, worin beide $R^1$ Cyclopentadienyl$^{\ominus}$ oder durch $C_1$-$C_4$-Alkyl substituiertes Cyclopentadienyl$^{\ominus}$ und $R^2$ und $R^3$ Reste der Formel V

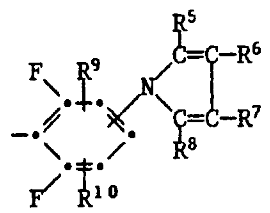

(V)

sind, worin $R^5$, $R^6$ und $R^8$ die zuvor angegebenen Bedeutungen haben und $R^9$ und $R^{10}$ unabhängig voneinander für H oder F stehen. $R^9$ und $R^{10}$ sind bevorzugt H.

In Formel V ist die Pyrrylgruppe bevorzugt in Orthostellung zu einem F-Atom gebunden. $R^9$ und $R^{10}$ bedeuten bevorzugt H.

Substituenten an $R^5$, $R^6$, $R^7$ und $R^8$ sind $C_2$-$C_8$-, bevorzugt $C_2$-$C_4$ Di-alkylamino oder -aminomethyl, z.B. Dimethyl-, Diethyl-, Di-n-propyl-, Di-n-Butyl- oder Methylethylamino und entsprechende -aminomethylreste; Bis[2-($C_1$-$C_4$-alkoxy)ethyl]amino oder -aminomethyl, z.B. Bis(2-Methoxyethyl)amino-aminomethyl oder Bis(2-Ethoxyethyl)-amino oder -aminomethyl; Morpholino; Morpholinylmethyl; Piperidino; Piperidinylmethyl; N-Methylpiperazino; N-Methylpiperazinylmethyl; Pyrrolidino; Pyrrolidinylmethyl; quaternäres $C_3$-$C_{10}$-Trialkyl-, bevorzugt $C_3$-$C_6$-Trialkylammonium oder -ammoniummethyl, z.B. Trimethyl-, Triethyl-, Dimethylethyl- oder Dimethylpropylammonium und entsprechende -ammoniummethylreste; $C_1$-$C_{12}$-, bevorzugt $C_1$-$C_4$-Alkoxy, z.B. Methoxy, Ethoxy, Propoxy und Butoxy;

$$\left(-OCH_2CH_2\right)_{l}-OC_1-C_{16}\text{-Alkyl},$$

worin 1 bevorzugt eine Zahl von 1 bis 3 bedeutet, z.B.

$$CH_3-O\left(-CH_2CH_2O\right)_{2}-;$$

1,3-Dioxolan-2-yl; 4-Methyl-1,3-dioxolan-2-yl, $-OCH_2CH_2O-$; $C_2$-$C_{12}$, bevorzugt $C_2$-$C_6$-Alkoxycarbonyl, z.B. Methoxy-, Ethoxy-, Propoxy- und Butoxycarbonyl; $C_2$-$C_{12}$-, bevorzugt $C_2$-$C_6$-Alkanoyloxy, z.B. Acetyl-, Propionyl- und Butyryloxy; $C_2$-$C_{12}$-, bevorzugt $C_2$-$C_6$-Alkanoyl, z.B. Acetyl, Propionyl und Butyryl; $C_1$-$C_{12}$-, bevorzugt $C_1$-$C_6$-Alkylthio, z.B. Methyl-, Ethyl-, Propyl- und Butylthio; Halogen, bevorzugt F, Cl und Br; Cyano; $SiR^4{}_3$ worin $R^4$ bevorzugt $C_1$-$C_6$-Alkyl bedeutet, z.B. Butyl, Propyl, Ethyl und besonders Methyl.

Unter den Substituenten sind solche aus der Gruppe $C_2$-$C_8$-Dialkylamino oder -aminomethyl, Bis(2-Methoxyethyl)amino oder -aminomethyl, Morpholino, Morpholinylmethyl, N-Methylpiperidino, N-Methylpiperidinylmethyl, $C_1$-$C_{12}$-Alkyldimethylammonium oder -ammoniummethyl, $C_1$-$C_{12}$-Alkoxy,

$$\left(-OCH_2CH_2\right)_{l}-O-C_1-C_{12}\text{-Alkyl}$$

mit l = 1 bis 3, 1,3-Dioxolan-2-yl, $-OCH_2CH_2O-$, $C_2$-$C_8$-Alkanoyloxy, $C_2$-$C_8$-Alkanoyl, $C_1$-$C_8$-Alkoxycarbonyl, Halogen, Cyano, Formyl, $C_1$-$C_4$-Alkylthio und Trimethylsilyl bevorzugt. Eine besonders bevorzugte Gruppe

von Substituenten sind ausgewählt aus $C_2$-$C_8$-Dialkylamino oder -aminomethyl, Bis(2-Methoxethyl)amino oder -aminomethyl, Morpholino, Morpholinylmethyl, $C_1$-$C_4$-Alkoxy, 1,3-Dioxolan-2-yl und Cyano. Die Reste $R^5$, $R^6$, $R^7$ und $R^8$ können ein- oder mehrfach, vorzugsweise ein-bis dreifach und besonders einfach substituiert sein. In einer bevorzugten Ausführungsform sind nur die Reste $R^5$ ,$R^6$, $R^7$ und $R^8$ in der Bedeutung von Alkyl wie definiert substituiert.

$R^5$, $R^6$, $R^7$ und $R^8$ in der Bedeutung von Alkyl enthalten bevorzugt 1 bis 12 und besonders 1 bis 8 C-Atome. Beispiele sind Methyl, Ethyl, sowie die Isomeren von Propyl, Butyl, Pentyl, Hexyl, Heptyl, Octyl, Nonyl, Decyl, Undecyl, Dodecyl, Tridecyl, Tetradecyl, Hexadecyl und Octadecyl. In der Bedeutung von Alkenyl enthalten $R^5$, $R^6$, $R^7$ und $R^8$ bevorzugt 2 bis 4 C-Atome. Beispiele sind Vinyl, Allyl, Crotonyl, 2-Methylprop-1-en-1-yl, But-1-en-1-yl, But-2-en-2-yl, But-2-en-1-yl, But-3-en-1-yl oder -2-yl, Pent-1-en-1-yl. $R^5$, $R^6$, $R^7$ und $R^8$ in der Bedeutung von Aryl sind insbesondere Phenyl. $R^5$, $R^6$, $R^7$ und $R^8$ in der Bedeutung von Aralkyl oder Alkaryl können z.B. Benzyl, Phenylethyl, Phenylpropyl, Methylphenyl, Ethylphenyl, Propylphenyl, Dimethylphenyl und Methylethylphenyl sein. $R^5$, $R^6$, $R^7$ und $R^8$ in der Bedeutung von Alkaralkyl können z.B. Methylbenzyl, Ethylbenzyl, Propylbenzyl, (Methylphenyl)ethyl oder Dimethylbenzyl sein. $R^5$, $R^6$, $R^7$ und $R^8$ in der Bedeutung von Cycloalkyl und Cycloalkenyl stellen besonders Cyclopentyl, Cyclopentenyl, Cyclohexyl oder Cyclohexenyl dar. $R^5$, $R^6$, $R^7$ und $R^8$ in der Bedeutung von Alkanoyl enthalten bevorzugt 2 bis 8, besonders 2 bis 6 C-Atome. Beispiele sind Acetyl, Propionyl, Butanoyl, Pentanoyl, Hexanoyl, Octanoyl und Dodecanoyl. $R^5$, $R^6$, $R^7$ und $R^8$ als $C_2$-$C_{12}$-Alkoxycarbonyl sind insbesondere $C_2$-$C_5$-Alkoxycarbonyl, wie z.B. Methoxycarbonyl, Ethoxycarbonyl oder Butoxycarbonyl. $R^5$, $R^6$, $R^7$ und $R^8$ können die Gruppen $-GeR^4{}_3$ und bevorzugt $-SiR^4{}_3$ bedeuten. $R^4$ stellt in diesen Gruppen bevorzugt $C_1$-$C_{12}$-, besonders $C_1$-$C_8$-und insbesondere $C_1$-$C_4$-Alkyl dar. Besonders bevorzugt ist die Gruppe $-Si(CH_3)_3$.

In einer bevorzugten Untergruppe stellen $R^5$, $R^6$, $R^7$ und $R^8$ unabhängig voneinander ein Wasserstoffatom oder unsubstituiertes oder substituiertes $C_1$-$C_{12}$-Alkyl, $C_2$-$C_5$-Alkenyl, $C_7$- oder $C_8$-Aralkyl oder -Alkaryl, Phenyl, 2-Furyl, $C_5$- oder $C_6$-Cycloalkyl, $C_5$- oder $C_6$-Cycloalkenyl, $C_2$-$C_8$-Alkanoyl $C_2$-$C_5$-Alkoxycarbonyl; -CHO oder $SiR^3{}_4$ dar, worin $R^4$ $C_1$-$C_8$-Alkyl ist.

In einer anderen bevorzugten Untergruppe stellen $R^5$, $R^6$, $R^7$ und $R^8$ unabhängig voneinander ein Wasserstoffatom, oder unsubstituiertes oder substituiertes $C_1$-$C_8$-Alkyl, $C_2$-$C_4$-Alkenyl, Benzyl, Phenyl, 2-Furyl, $C_5$-oder $C_6$-Cycloalkyl, $C_2$-$C_6$-Alkanoyl, $C_2$-$C_5$-Alkoxycarbonyl, -CHO oder $SiR^4{}_3$ dar, worin $R^4$ $C_1$-$C_4$-Alkyl ist.

In einer weiteren bevorzugten Untergruppe bedeuten $R^5$, $R^6$, $R^7$ und $R^8$ unabhängig voneinander ein Wasserstoffatom oder unsubstituiertes oder substituiertes $C_1$-$C_8$-Alkyl, $C_2$-$C_4$-Alkenyl, Phenyl, 2-Furyl oder $SiR^4{}_3$, worin $R^4$ $C_1$-$C_4$-Alkyl ist.

In einer bevorzugten Ausführungsform stehen in Formel IV $R^6$ oder $R^6$ und $R^7$ für ein Wasserstoffatom.

Einige Beispiele für Verbindungen der Formel I sind:

Bis(cyclopenadienyl)-bis[2,6-difluor-3-(2,5-dimethyl-pyrr-1-yl)phenyl]-titan
Bis(trimethylsilylcyclopentadienyl)-bis[2,6-difluor-3-(2,5-dimethylpyrr-1-yl)phenyl]-titan
Bis(methylcyclopentadienyl)-bis[2,6-difluor-3-(2,5-dimethylpyr-1-yl)phenyl]-titan
Bis(cyclopentadienyl)-bis[2,6-difluor-3-(2-isopropyl-5-methylpyrr-1-yl)phenyl]-titan
Bis(cyclopentadienyl)-bis[2,6-difluor-3-(2-(1-ethylpentyl)-5-methylpyrr-1-yl)phenyl]-titan
Bis(cyclopentadienyl)-bis[2,6-difluor-3-(2-(2-methoxyethyl)-5-methylpyrr-1-yl)phenyl]-titan
Bis(cyclopentadienyl)-bis[2,6-difluor-3-(5-methyl-2-phenylpyrr-1-yl) phenyl]-titan
Bis(cyclopentadienyl)-bis[2,6-difluor-3-(2-(2-furyl)-5-methylpyrr-1-yl)phenyl]-titan
Bis(cyclopentadienyl)-bis[2,6-difluor-3-(2,5-bis(2-methylprop-1-enyl)pyrr-1-yl)phenyl]-titan
Bis(cyclopentadienyl)-bis[2,6-difluor-3-(2,5-dimethyl-3-(2-methylprop-1-enyl)pyrr-1-yl)phenyl]-titan
Bis(cyclopentadienyl)-bis[2,6-difluor-3-(2,5-dimethyl-3-(trimethylsilyl)pyrr-1-yl)phenyl]-titan
Bis(cyclopentadienyl)-bis[2,6-difluor-3-(pyrr-1-yl)phenyl]-titan
Bis(cyclopentadienyl)-bis[2,6-difluor-3-(2,5-bis(dimethylaminomethyl)-pyrr-1-yl)phenyl]-titan
Bis(cyclopentadienyl)-bis[2,6-difluor-3-(2,5-bis(bis(2-methoxyethyl)-aminomethyl)pyrr-1-yl)phenyl]-titan
Bis(cyclopentadienyl)-bis[2,6-difluor-3-(2,5-dimethyl-3-(dimethylaminomethyl)pyrr-1-yl)phenyl]-titan
Bis(cyclopentadienyl)-bis[2,6-difluor-3-(2,5-dimethyl-3,4-bis(dimethylaminomethyl)-pyrr-1-yl)phenyl]-titan
Bis(cyclopentadienyl)-bis[2,6-difluor-3-(2,5-dimethyl-3-(trimethylammoniummethyl)pyrr-1-yl)phenyl]-titanchlorid
Bis(cyclopentadienyl)-bis[2,6-difluor-3-(2,5-dimethyl-3-((bis(2-methoxyethyl)amino)methyl)pyrr-1-yl)phenyl]-titan
Bis(cyclopentadienyl)-bis[2,6-difluor-3-(2,5-dimethyl-3-(morpholino)-methyl)pyrr-1-yl)phenyl]-titan
Bis(methylcyclopentadienyl)-bis[2,6-difluor-3-(2,5-dimethyl-3-((dibutylamino)methyl)pyrr-1-yl)phenyl]-titan
Bis(cyclopentadienyl)-bis[2,6-difluor-3-(2,5-dimethyl-3-((ethyloxy)-methyl)pyrr-1-yl)phenyl)-titan

Bis(cyclopentadienyl)-bis[2,3,5,6-tetrafluor-4-(2,5-dimethyl-3-((4-methyl-1-piperazinyl)methyl)pyrr-1-yl)-phenyl]-titan
Bis(cyclopentadienyl)-bis[2,3,5,6-tetrafluor-4-(2-(1-ethylpentyl)-5-methyl-pyrr-1-yl)phenyl]-titan
Bis(cyclopentadienyl)-bis[2,6-difluor-3-(2,5-dimethyl-3-((dimethoxy)-methyl)pyrr-1-yl)phenyl]-titan
Bis(methylcyclopentadienyl)-bis[2,6-difluor-3-(5-isopropyl-2-methyl-3-((diethoxy)methyl)pyrr-1-yl)phenyl]-titan
Bis(cyclopentadienyl)-bis[2,6-difluor-3-(2,5-dimethyl-3-(1,3-dioxolan-2-yl)pyrr-1-yl)phenyl]-titan
Bis(cyclopentadienyl)-bis[2,6-difluor-3-(2-(4-methyl-1,3-dioxolan-2-yl)pyrr-1-yl)phenyl]-titan
Bis(cyclopentadienyl)-bis[2,6-difluor-3-((1,1-ethylendioxy-ethyl)pyrr1-yl)phenyl]-titan
Bis(cyclopentadienyl)-bis[2,6-difluor-3-(2,5-bis(2,2-diethoxyethyl)pyrr1-yl)phenyl]-titan
Bis(cyclopentadienyl)-bis[2,6-difluor-3-(2-methyl-3-(dimethoxy-methyl)-5-(2,2-dimethoxy-ethyl)pyrr-1-yl)-phenyl]-titan
Bis(cyclopentadienyl)-bis[2,6-difluor-3-(2,5-dimethyl-3-(diethoxymethyl)pyrr-1-yl)phenyl]-titan
Bis(cyclopentadienyl)-bis[2,6-difluor-3-(5-methyl-2-(2,2-dimethoxyethyl)pyrr-1-yl)phenyl]-titan
Bis(methylcyclopentadienyl)-bis[2,6-difluor-3-(2,5-dimethyl-3-propylthio-pyrr-1-yl)phenyl]-titan
Bis(trimethylsilylcyclopentadienyl)-bis[2,6-difluor-3-(2,5-dimethyl-3-trimethylsilyl-pyrr-1-yl)phenyl]-titan
Bis(cyclopentadienyl)-bis[2,6-difluor-3-(2,5-diethoxymethyl-pyrr-1-yl)phenyl]-titan
Bis(cyclopentadienyl)-bis[2,6-difluor-3-(2-methyl-4,6-dihydro-1H-furo-[3,4-b]pyrr-1-yl)phenyl]-titan
Bis(cyclopentadienyl)-bis[2,6-difluor-3-(4,6-dimethyl-3,5-dihydro-1H-furo[3,4-c]pyrr-5-yl)phenyl]-titan
Bis(cyclopentadienyl)-bis[2,6-difluor-3-(2,5-dimethyl-1,4,5,6-tetrahydro-pyrrolo[3,4-b]pyrr-1-yl)phenyl]-titan
Bis(cyclopentadienyl)-bis[2,6-difluor-3-(1,3,5-trimethyl-2,4,5,6-tetra-hydro-pyrrolo[3,4-c]pyrr-2-yl)phenyl]-titan
Bis(cyclopentadienyl)-bis[2,6-difluor-3-(1,4,5,7-tetrahydro-3H-difuro[3,4-b:3',4'-d]pyrr-4-yl)phenyl]-titan
Bis(cyclopentadienyl)-bis[2,6-difluor-3-(2,6-dimethyl-2,3,4,5,6,7-hexahydro-1H-dipyrrolo[3,4-b:3',4'-d]pyrr-4-yl)phenyl]-titan
Bis(cyclopentadienyl)-bis[2,6-difluor-3-(6-methyl-1,3,4,5,6,7-hexahydro-furo[3,4-b]pyrrolo[3,4-d]pyrr-4-yl)-phenyl]-titan
Bis(cyclopentadienyl)-bis[2,6-difluor-3-(2-isobutyl-5-methyl-pyrr-1-yl)phenyl]-titan
Bis(cyclopentadienyl)-bis[2,6-difluor-3-(2-(1-ethylpentyl)-5-methylpyrr-1-yl)phenyl]-titan
Bis(cyclopentadienyl)-bis[2,6-difluor-3-(2-(cyclohex-3-enyl)-5-methylpyrr-1-yl)phenyl]-titan
Bis(cyclopentydienyl)-bis[2,6-difluor-3-(2-(6-methyl-6-methoxy-heptyl)-5-methyl-pyrr-1-yl)phenyl]-titan
Bis(cyclopentadienyl)-bis[2,6-difluor-3-(2-(1,1-dimethyl-2-methoxyethyl)-5-methyl-pyrr-1-yl)phenyl]-titan
Bis(cyclopentadienyl)-bis[2,6-difluor-3-methyl-4-(2,5-dimethyl-3-(2-methylprop-1-enyl)-pyrr-1-yl)phenyl]-titan
Bis(cyclopentadienyl)-bis[2,6-difluor-5-methyl-3-(2,5-dimethyl-3-(ethoxymethyl)pyrr-1-yl)phenyl]-titan
Bis(cyclopentadienyl)-bis[2,6-difluor-5-chlor-3-(5-methyl-2-(1-ethylpentyl)pyrr-1-yl)phenyl]-titan.
Bis(cyclopentadienyl)-bis[2,4,6-trifluor-3-(pyrr-1-yl)phenyl]-titan
Bis(cyclopentadienyl)-bis[2,5,6-trifluor-3-(2,5-dimethylpyrr-1-yl)-phenyl]-titan
Bis(cyclopentadienyl)-bis[4-brom-2,6-difluor-3-(pyrr-1-yl)phenyl]-titan
Bis(cyclopentadienyl)-bis[2,4,5,6-tetrafluor-3-(2,5-dimethylpyrr-1-yl)-phenyl]-titan
Bis(cyclopentadienyl)-bis[2,4,5,6-tetrafluor-3-(pyrr-1-yl)phenyl]-titan
Bis(cyclopentadienyl)-bis[4-methyl-2,5,6-trifluor-3-(pyrr-1-yl)phenyl]-titan
Bis(cyclopentadienyl)-bis[2,6-difluor-3-(2,5-diethylpyrr-1-yl)phenyl]-titan
Bis(cyclopentadienyl)-bis[2,6-difluor-3-(2,5-dimethyl-3-ethoxycarbonylpyrr-1-yl)phenyl]-titan
Bis(cyclopentadienyl)-bis[2,6-difluor-3-(2,3,4,5-tetramethylpyrr-1-yl)-phenyl]-titan
Bis(cyclopentadienyl)-bis[2,6-difluor-3-(2,3,5-trimethylpyrr-1-yl)phenyl]-titan
Bis(cyclopentadienyl)-bis[2,6-difluor-3-(2,4-dimethylpyrr-1-yl)phenyl]-titan
Bis(cyclopentadienyl)-bis[2,6-difluor-3-(3,4-dimethylpyrr-1-yl)phenyl]-titan
Bis(cyclopentadienyl)-bis[2,6-difluor-3-(2-(2,5-dioxahexyl)-5-methylpyrr-1-yl)phenyl]-titan
Bis(cyclopentadienyl)-bis[2,6-difluor-3-(2-(2,5-dioxanonyl)-5-methylpyrr-1-yl)phenyl]-titan
Bis(cyclopentadienyl)-bis[2,6-difluor-3-(4,5,6,7-tetrahydro-2H-isoindol-2-yl)phenyl]-titan
Bis(cyclopentadienyl)-bis[2,6-difluor-3-(2H-isoindol-2-yl)phenyl]-titan
Bis(cyclopentadienyl)-bis[2,6-difluor-3-(1,3-diphenyl-1H-isoindol-2-yl)-phenyl]-titan
Bis(cyclopentadienyl)-bis[2,6-difluor-3-(1,4,5,6-tetrahydrocyclopenta-[b]pyrr-1-yl)phenyl]-titan
Bis(cyclopentadienyl)-bis[2,6-difluor-3-(1,4,5,6-tetrahydro-2-phenylcyclopenta[b]pyrr-1-yl)phenyl)-titan
Bis(cyclopentadienyl)-bis[2,6-difluor-3-(1,4,5,6-tetrahydro-2-methylcyclopenta[b]pyrr-1-yl)phenyl]-titan
Bis(cyclopentadienyl)-bis[2,6-difluor-3-(4,5,6,7-tetrahydro-1H-indol1-yl)phenyl]-titan
Bis(cyclopentadienyl)-bis[2,6-difluor-3-(4,5,6,7-tetrahydro-2-phenyl-1H-indol-1-yl)phenyl]-titan
Bis(cyclopentadienyl)-bis[2,6-difluor-3-(4,5,6,7-tetrahydro-2-methyl-1H-indol-1-yl)phenyl]-titan
Bis(cyclopentadienyl)-bis[2,6-difluor-3-(2,3,4,5,6,7-hexahydro-1H-dicyclopenta[b,d]pyrr-1-yl)phenyl]-titan

Bis(cyclopentadienyl)-bis[2,6-difluor-3-(2,3,4,5,6,7,8,9-octahydro-1H-carbazol-9-yl)phenyl]-titan
Bis(cyclopentadienyl)-bis[2,6-difluor-3-(2,3,4,9-tetrahydro-1H-carbazol9-yl)phenyl]-titan
Bis(cyclopentadienyl)-bis[2,6-difluor-3-(2,3,4,9-tetrahydro-6-methoxy-1H-carbazol-9-yl)phenyl]-titan
Bis(cyclopentadienyl)-bis[2,6-difluor-3-(9H-carbazol-9-yl)phenyl]-titan
Bis(cyclopentadienyl)-bis[2,6-difluor-4-(9H-carbozol-9-yl)phenyl]-titan
Bis(cyclopentadienyl)-bis[2,3,5,6-tetrafluor-4-(9H-carbazol-9-yl)phenyl]-titan
Bis(cyclopentadienyl)-bis[2,4,6-trifluor-5-methyl-3-(9H-carbazol-9-yl)-phenyl]-titan
Bis(cyclopentadienyl)-bis[2,5,6-trifluor-3-(9H-carbazol-9-yl-phenyl]-titan
Bis(cyclopentadienyl)-bis[2,6-difluor-3-(1H-indol-1-yl)phenyl]-titan
Bis(cyclopentadienyl)-bis[2,6-difluor-3-(2-methyl-1H-indol-1-yl)phenyl]-titan
Bis(cyclopentadienyl)-bis[2,6-difluor-3-(2-methoxycarbonyl-1H-indol-1-yl)phenyl]-titan
Bis(cyclopentadienyl)-bis[2,6-difluor-3-(2-ethoxycarbonyl-1H-indol-1-yl)phenyl]-titan
Bis(cyclopentadienyl)-bis[2,6-difluor-3-(5-hydroxy-2-methyl-1H-indol-1-yl)phenyl]-titan
Bis(cyclopentadienyl)-bis[2,6-difluor-3-(5-methoxy-2-methyl-1H-indol-1-yl)phenyl-titan
Bis(cyclopentadienyl)-bis[2,6-difluor-3-(5-acetoxy-2-methyl-1H-indol-1-yl)phenyl]-titan
Bis(cyclopentadienyl)-bis[2,6-difluor-3-(5-hydroxy-3-methyl-1H-indol-1-yl)phenyl]-titan
Bis(cyclopentadienyl)-bis[2,6-difluor-3-(2,3-dimethyl-1H-indol-1-yl)-phenyl]-titan
Bis(cyclopentadienyl)-bis[2,3,5,6-tetrafluor-4-(2,3-dimethyl-1H-indol-1-yl)phenyl]-titan

Die Herstellung der Titanocene der Formel I kann nach bekannten oder analogen Verfahren erfolgen, indem man 1 Mol einer Verbindung der Formel VI

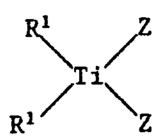

(VI),

worin $R^1$ die angegebene Bedeutung hat und Z für Halogen, besonders Chlor, steht, mit einem Mol $LiR^2$ und mit einem Mol $LiR^3$ oder mit 1 Mol $Li_2QYQ$ umsetzt, wobei $R^2$, $R^3$ und QYQ die zuvor angegebenen Bedeutungen haben, und danach die Verbindungen der Formel I in an sich bekannter Weise isoliert.

Die bekannten Verfahren sind z.B. in J. Organometal. Chem., 2 (1964) 206-212, J. Organometal. Chem., 4 (1965) 445-446 und in der EP-A-0 122 223 beschrieben.

Die Ausgangsverbindungen der Formel VI, in denen Z besonders für Chlor steht, sind bekannt oder können nach analogen Verfahren durch die Umsetzung von $TiCl_4$ mit Natriumverbindungen $NaR^1$ erhalten werden. Die Lithiumverbindungen $LiR^2$ und $Li_2QYQ$ sind neu. Sie können nach an sich bekannten Verfahren durch die Umsetzung von z.B. Lithiumbutyl mit Verbindungen der Formel VII hergestellt werden.

Ein weiterer Gegenstand der Erfindung sind Verbindungen der Formel VII

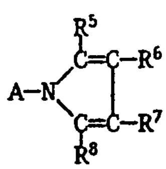

(VII),

worin A einen 6-gliedrigen carbocyclischen oder 5- oder 6-gliedrigen heterocyclischen aromatischen Ring, der mindestens ein Fluoratom, in Orthostellung hierzu ein Wasserstoffatom oder ein Halogenatom und gegebenenfalls weitere Substituenten enthält, oder A einen Rest der Formel

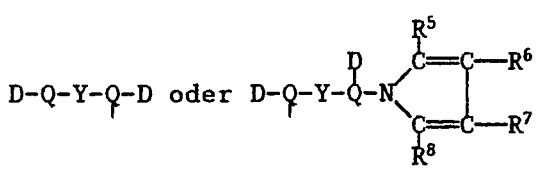

bedeutet, worin D für ein in Orthostellung zu Y gebundenes Wasserstoffatom oder Halogenatom steht, Q einen carbocyclischen aromatischen Ring bedeutet, der in Orthostellung zur D-Gruppe jeweils durch ein

Fluoratom substituiert ist und Q weitere Substituenten enthalten kann, und Y, $R^5$, $R^6$, $R^7$ und $R^8$ die zuvor angegebenen Bedeutungen haben, mit Ausnahme von 3,4-Dimethyl-1-(pentafluorphenyl)pyrrol.

Für A, $R^5$, $R^6$, $R^7$, $R^6$, Q und Y gelten sinngemäss die gleichen Ausführungsformen und Bevorzugungen wie sie zuvor für $R^2$ bzw. $R^2$ und $R^3$ zusammen und für den Rest der Formel IV beschrieben sind.

Der Rest A in der Bedeutung eines 6-gliedrigen carbocyclischen oder 5-oder 6-gliedrigen heterocyclischen aromatischen Ringes enthält vorzugsweise in Orthostellung zum Wasserstoff- bzw. Halogenatom ein weiteres Fluoratom. Das Halogenatom ist bevorzugt aus F, Cl oder Br ausgewählt. Beim aromatischen Ring handelt es sich vorzugsweise um einen Phenylring. In Orthostellung zum Fluoratom bzw. zu Y ist insbesondere ein Wasserstoffatom gebunden. Die Pyrrolgruppe ist vorzugsweise in Orthostellung zu einem F-Atom gebunden. Es wurde gefunden, dass bei solchen Verbindungen überraschend das zum F-Atom benachbarte H-Atom direkt durch Lithium ersetzt werden kann. Eine bevorzugte Gruppe von Verbindungen sind solche der Formel VIIa

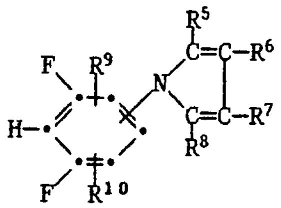

(VIIa),

worin $R^9$ und $R^{10}$ unabhängig voneinander für H oder F, besonders für H, stehen, und $R^5$, $R^6$, $R^7$ und $R^8$ die zuvor angegebene Bedeutung haben. Die Pyrrolgruppe ist vorzugsweise in Orthostellung zum Fluoratom gebunden.

Die Herstellung der Verbindungen der Formel VII kann nach an sich bekannten Verfahren erfolgen. 3,4-Dimethyl-1-(pentafluorphenyl)-pyrrol ist von U. Jäger et al. in Chem. Ber. 119, S. 3405 - 3410 (1986) beschrieben. Eine allgemeine Herstellungsmöglichkeit ist die Umsetzung von Aminen der Formel A-$NH_2$ mit gegebenenfalls substituierten 1,4-Diketonen, 1,4-Dialdehyden oder 1,4-Ketoaldehyden (Paal-Knorr-Synthese). Diese Reaktion ist z.B.in Comprehensive Organic Chemistry, Vol. 4, 299 und in J. Chem. Soc., 1952, 1467 beschrieben. Die Amine und Diketone und Dialdehyde sind bekannt, teilsweise kommerziell erhältlich oder nach bekannten Verfahren herstellbar.

Verbindungen der Formel VII, worin $R^5$ und $R^8$ H bedeuten, erhält man auch durch Umsetzung von Aminen A-$NH_2$ mit gegebenenfalls substituiertem 2,5-Dimethoxytetrahydrofuran oder 2,5-Diacetoxytetrahydrofuran, vgl. G. Stefencich et al, Farmaco Ed. Sc. 40, 237 - 248 (1985) und Houben-Weyl, Band 6/3, 730.

Weitere Synthesen von Verbindungen der Formel VII aus den Aminen A-$NH_2$ sind die Umsetzung mit 2,2'-(Ethane-1,2-diyl)-bis-1,3-dioxolanen, die Umsetzung mit 2-Alkin-1,4-diolen oder die Umsetzung mit $\alpha$-Haloketonen und $\beta$-Ketoestern (Hantzsch-Synthese), siehe Comprehensive Organic Chemistry, Vol. 4, 298.

Verbindungen der Formel VII mit Indol-Struktur können z.B. nach der Neuitzescu-Synthese oder nach der Bischler-Synthese hergestellt werden (Comprehensive Organic Chemistry, Vol. 4, 461 und 462). Verbindungen der Formel VII mit Isoindol-Struktur können z.B. aus dem entsprechenden Phthalimiden durch Reduktion mit komplexen Metallhydriden hergestellt werden (Houben-Weyl, Band 4/1d, 258). Verbindungen der Formel VII mit Carbazol-Struktur können z.B. nach der Methode von Dunlop und Tucker (J. Chem. Soc. 1939, 1945) hergestellt werden.

In unsubstituierte oder teilweise substituierte Pyrrolderivate der Formel VII können nachträglich Substituenten eingeführt werden. Mit Trifluormethansulfonsäuresilylestern können Silylgruppen eingeführt werden [G. Simchen, Tetrahedron 42(5), S. 1299 (1986)]. Ferner kann mittels $POCl_3$/DMF formyliert werden [G.F. Smith, J. Chem. Soc., 1954, 3842]. Mittels ROCl oder $(RCO)_2O$ und $AlCl_3$ kann acyliert werden, vgl. J. Rockach et al., Tetrahedron Letters, 22, 4901 (1981) und M. Kakushima et al. J. Org. Chem., 48, 3214 (1983). Die Formylgruppe oder Acylgruppe kann im Rahmen der Erfindung weiter derivatisiert werden; z.B. kann mittels Wittig-Reaktion eine $C_2$-$C_5$-Alkenylgruppe gebildet werden.

Die Herstellung der Metallocene der Formel I erfolgt im allgemeinen in Gegenwart inerter Lösungsmittel wie z.B. Kohlenwasserstoffen oder Ethern, bei Temperaturen von -30 bis -100 °C, vorzugsweise -60 bis -90 °C und unter Schutzgasatmosphäre. In einer Ausführungsform des Verfahrens wird zunächst $LiR^2$ bzw. $LiR^3$ durch Umsetzung der entsprechenden Halogenide in einem Ether als Lösungsmittel, z.B. Tetrahydrofuran, mit Lithiumbutyl bei Temperaturen um -78 °C hergestellt. Zu der gekühlten Reaktionsmischung gibt man dann das entsprechende Titanocendihalogenid, entfernt die Kühlung und lässt auf Raumtemperatur erwärmen. Die Reaktionsmischung wird dann, gegebenenfalls nach Zugabe von Lösungsmitteln, filtriert und aus der Lösung durch Ausfällen oder Verdampfen des Lösungsmittels das erfindungsgemässe Titanocen

isoliert.

Es handelt sich im allgemeinen um kristalline meist orange-gefärbte Verbindungen, die sich durch eine hohe thermische Stabilität auszeichnen und sich erst bei hohen Temperaturen zersetzen. Auch unter Lufteinwirkung sowie unter Einwirkung von Wasser wird keine Zersetzung beobachtet. Die Verbindungen können in härtbaren Zusammensetzungen auch in höheren Mengen gelöst werden, und bieten daher wertvolle anwendungstechnische Eigenschaften. Die Verbindungen sind auch in Lösungsmitteln gut löslich, und können in Form von Lösungen in härtbare Zuammensetzungen eingearbeitet werden, wonach das Lösungsmittel gegebenenfalls entfernt wird.

Die Verbindungen sind dunkellagerstabil und können ohne Schutzgas gehandhabt werden. Sie eignen sich alleine hervorragend als sehr wirksame Photoinitiatoren für die lichtinduzierte Polymerisation ethylenisch ungesättigter Verbindungen. Sie zeichnen sich hierbei durch eine sehr hohe Lichtempfindlichkeit und Wirksamkeit über einen grossen Wellenlängenbereich von ca. 200 nm (UV-Licht) bis etwa 600 nm aus. Ferner vermögen die Titanocene auch wirksam die Polymerisation unter dem Einfluss von Wärme zu initiieren, wobei ein Erwärmen auf 170 °C bis 240 °C zweckmässig ist. Selbstverständlich kann auch Lichteinwirkung und Erwärmung zur Polymerisation benutzt werden, wobei eine Erwärmung nach der Belichtung tiefere Temperaturen, z.B. 80-150 °C, zur Polymerisation erlaubt. Die Lichtempfindlichkeit ist überraschend höher als bei Pyrrolidinderivaten.

Ein weiterer Gegenstand vorliegender Erfindung ist eine durch Strahlung polymerisierbare Zusammensetzung, enthaltend (a) mindestens eine nichtflüchtige, monomere, oligomere oder polymere Verbindung mit mindestens einer polymerisierbaren ethylenisch ungesättigten Doppelbindung und (b) mindestens ein Titanocen der Formel I als Photoinitiator.

Die Zusammensetzungen können weitere von (b) verschiedene Photoinitiatoren (c), z.B. solche vom Typ der Benzoinalkylether, Benzophenone, Benzilketale, 4-Aroyl-1,3-dioxolane, Dialkoxyacetophenone, α-Hydroxy- oder α-Aminoacetophenone, α-Hydroxycycloalkylphenylketone oder Mischungen davon enthalten. Der Vorteil besteht darin, dass man geringere Mengen der erfindungsgemässen Titanocene verwenden kann und trotzdem gleiche oder verbesserte Lichtempfindlichkeiten erzielen kann. Das Gewichtsverhältnis dieser Komponenten (c):(b) kann z.B. von 1:1 bis 30:1, bevorzugt 5:1 bis 15:1 betragen.

Die Zusatzmenge der erfindungsgemässen Titanocene richtet sich im wesentlichen nach wirtschaftlichen Gesichtspunkten, deren Löslichkeiten und nach der gewünschten Empfindlichkeit. Im allgemeinen werden 0,01 bis 20, vorzugsweise 0,05-10 und besonders 0,1 bis 5 Gew.% verwendet, bezogen auf die Komponente (a).

Als Komponente (a) kommen solche ethylenisch ungesättigten monomeren, oligomeren und polymeren Verbindungen in Frage, die durch Photopolymerisation zu höhermolekularen Produkten reagieren und hierbei ihre Löslichkeit verändern.

Besonders geeignet sind z.B. Ester von ethylenisch ungesättigten Carbonsäuren und Polyolen oder Polyepoxiden, und Polymere mit ethylenisch ungesättigten Gruppen in der Kette oder in Seitengruppen, wie z.B. ungesättigte Polyester, Polyamide und Polyurethane und Copolymere hiervon, Polybutadien und Butadien-Copolymere, Polyisopren und Isopren-Gopolymere, Polymere und Copolymere mit (Meth)-Acrylgruppen in Seitenketten, sowie Mischungen von einem oder mehreren solcher Polymerer.

Beispiele für ungesättigte Carbonsäuren sind Acrylsäure, Methacrylsäure, Crotonsäure, Itaconsäure, Zimtsäure, ungesättigte Fettsäuren wie Linolensäure oder Oelsäure. Bevorzugt sind Acryl- und Methacrylsäure.

Als Polyole sind aromatische und besonders aliphatische und cycloaliphatische Polyole geeignet. Beispiele für aromatische Polyole sind Hydrochinon, 4,4'-Dihydroxydiphenyl, 2,2-Di(4-hydroxyphenyl)-propan, sowie Novolake und Resole. Beispiele für Polyepoxide sind solche auf der Basis der genannten Polyole, besonders der aromatischen Polyole und Epichlorhydrin. Ferner sind auch Polymere oder Copolymere, die Hydroxylgruppen in der Polymerkette oder in Seitengruppen enthalten, wie z.B. Polyvinylalkohol und Copolymere davon oder Polymethacrylsäurehydroxyalkylester oder Copolymere davon, als Polyole geeignet. Weitere geeignete Polyole sind Oligoester mit Hydroxylendgruppen.

Beispiele für aliphatische und cycloaliphatische Polyole sind Alkylendiole mit bevorzugt 2 bis 12 C-Atomen, wie Ethylenglykol, 1,2- oder 1,3-Propandiol, 1,2-, 1,3 oder 1,4-Butandiol, Pentandiol, Hexandiol, Octandiol, Dodecandiol, Diethylenglykol, Triethylenglykol, Polyethylenglykole mit Molekulargewichten von bevorzugt 200 bis 1500, 1,3-Cyclopentandiol, 1,2-, 1,3- oder 1,4-Cyclohexandiol, 1,4-Dihydroxymethylcyclohexan, Glycerin, Tris-(β-hydroxyethyl)amin, Trimethylolethan, Trimethylolpropan, Pentaerythrit, Dipentaerythrit und Sorbit.

Die Polyole können teilweise oder vollständig mit einer oder verschiedenen ungesättigten Carbonsäuren verestert sein, wobei in Teilestern die freien Hydroxylgruppen modifiziert, z.B. verethert oder mit anderen Carbonsäuren verestert sein können.

Beispiele für Ester sind:
Trimethylolpropantriacrylat, Trimethylolethantriacrylat, Trimethylolpropantrimethacrylat, Trimethylolethantrimethacrylat, Tetramethylenglykoldimethacrylat, Triethylenglykoldimethacrylat, Tetraethylenglykoldiacrylat, Pentaerythritdiacrylat, Pentaerythrittriacrylat, Pentaerythrittetraacrylat, Dipentaerythritdiacrylat, Dipentaerythrittriacrylat, Dipentaerythrittetraacrylat, Dipentaerythritpentaacrylat, Dipentaerythrithexaacrylat,Tripentaerythritoctaacrylat, Pentaerythritdimethacrylat, Pentaerythrittrimethacrylat, Dipentaerythritdimethacrylat, Dipentaerythrittetramethacrylat, Tripentaerythritoctamethacrylat, Pentaerythritdiitaconat, Dipentaerythrittrisitaconat, Dipentaerythritpentaitaconat, Dipentaerythrithexaitaconat, Ethylenglykoldimethacrylat, 1,3-Butandioldiacrylat, 1,3-Butandioldimethacrylat, 1,4-Butandioldiitaconat, Sorbittriacrylat, Sorbittetraacrylat, Pentaerythrit-modifiziert-triacrylat, Sorbittetramethacrylat, Sorbitpentaacrylat, Sorbithexaacrylat, Oligoesteracrylate und -methacrylate, Glyzerindi- und -triacrylat, 1,4-Cyclohexandiacrylat, Bisacrylate und Bismethacrylate von Polyethylenglykol mit Molekulargewicht von 200-1500, oder Gemische davon.

Als Komponente (a) sind auch die Amide gleicher oder verschiedener ungesättigter Carbonsäuren von aromatischen, cycloaliphatischen und aliphatischen Polyaminen mit bevorzugt 2 bis 6, besonders 2 bis 4 Aminogruppen geeignet. Beispiele für solche Polyamine sind Ethylendiamin, 1,2- oder 1,3-Propylendiamin, 1,2-, 1,3- oder 1,4-Butylendiamin, 1,5-Pentylendiamin, 1,6-Hexylendiamin, Octylendiamin, Dodecylendiamin, 1,4-Diaminocyclohexan, Isophorondiamin, Phenylendiamin, Bisphenylendiamin, Di-$\beta$-aminoethylether, Diethylentriamin, Triethylentetramin, Di-($\beta$-aminoethoxy)- oder Di($\beta$-aminopropoxy)ethan. Weitere geeignete Polyamine sind Polymere und Copolymere mit Aminogruppen in der Seitenkette und Oligoamide mit Aminoendgruppen.

Beispiele für solche ungesättigten Amide sind: Methylen-bis-acrylamid, 1,6-Hexamethylen-bis-acrylamid, Diethylentriamin-tris-methacrylamid, Bis(methacrylamidopropoxy)-ethan, $\beta$-Methacrylamidoethylmethacrylat,N[($\beta$-Hydroxyethoxy)ethyl]-acrylamid.

Geeignete ungesättigte Polyester und Polyamide leiten sich z.B. von Maleinsäure und Diolen oder Diaminen ab. Die Maleinsäure kann teilweise durch andere Dicarbonsäuren ersetzt sein. Sie können zusammen mit ethylenisch ungesättigten Comonomeren, z.B. Styrol, eingesetzt werden. Die Polyester und Polyamide können sich auch von Dicarbonsäuren und ethylenisch ungesättigten Diolen oder Diaminen ableiten, besonders von längerkettigen mit z.B. 6 bis 20 C-Atomen. Beispiele für Polyurethane sind solche, die aus gesättigten oder ungesättigten Diisocyanaten und ungesättigten bzw. gesättigten Diolen aufgebaut sind.

Polybutadien und Polyisopren und Copolymere davon sind bekannt. Geeignete Comonomere sind z.B. Olefine wie Ethylen, Propen, Buten, Hexen, (Meth)Acrylate, Acrylnitril, Styrol oder Vinylchlorid. Polymere mit (Meth)Acrylatgruppen in der Seitenkette sind ebenfalls bekannt. Es kann sich z.B. um Umsetzungsprodukte von Epoxidharzen auf Novolakbasis mit (Meth)Acrylsäure handeln, um Homo- oder Copolymere des Polyvinylalkohols oder deren Hydroxyalkylderivaten, die mit (Meth)Acrylsäure verestert sind, oder um Homo- und Copolymere von (Meth)Acrylaten, die mit Hydroxyalkyl(meth)acrylaten verestert sind.

Die photopolymerisierbaren Verbindungen können alleine oder in beliebigen Mischungen eingesetzt werden. Bevorzugt werden Gemische von Polyol(Meth)Acrylatenverwendet.

Den erfindungsgemässen Zusammensetzungen können auch Bindemittel zugesetzt werden, was besonders zweckmässig ist, wenn es sich bei den photopolymerisierbaren Verbindungen um flüssige oder viskose Substanzen handelt. Die Menge des Bindemittels kann z.B. 5-95, vorzugsweise 10-90 und besonders 50-90 Gew.% betragen, bezogen auf die gesamte Zusammensetzung. Die Wahl des Bindemittels erfolgt je nach dem Anwendungsgebiet und hierfür geforderter Eigenschaften wie Entwickelbarkeit in wässrigen und organischen Lösungsmittelsystemen, Adhäsion auf Substraten und Sauerstoffempfindlichkeit.

Geeignete Bindemittel sind z.B. Polymere mit einem Molekulargewicht von etwa 5000-2 000 000, bevorzugt 10 000 bis 1 000 000. Beispiele sind: Homo- und copolymere Acrylate und Methacrylate, z.B. Copolymere aus Methylmethacrylat/Ethylacrylat/Methacrylsäure, Poly(methacrylsäurealkylester), Poly(acrylsäurealkylester); Celluloseester und -ether wie Celluloseacetat, Celluloseacetatbutyrat, Methylcellulose, Ethylcellulose; Polyvinylbutyral, Polyvinylformal, cyclisierter Kautschuk, Polyether wie Polyethylenoxid, Polypropylenoxid, Polytetrahydrofuran; Polystyrol, Polycarbonat, Polyurethan, chlorierte Polyolefine, Polyvinylchlorid, Copolymere aus Vinylchlorid/Vinylidenchlorid, Copolymere von Vinylidenchlorid mit Acrylnitril, Methylmethacrylat und Vinylacetat, Polyvinylacetat, Copoly(ethylen/vinylacetat), Polyamide wie Polycaprolactam und Poly(hexamethylenadipamid), Polyester wie Poly(äthylenglykolterephthalat) und Poly(hexamethylenglykolsuccinat).

Die erfindungsgemässen Zusammensetzungen eignen sich als Beschichtungsmittel für Substrate aller Art, z.B. Holz, Papier, Keramik, Kunststoffe, wie Polyester- und Celluloseacetatfilme, und Metalle, wie Kupfer und Aluminium, bei denen durch Photopolymerisation eine Schutzschicht oder eine photographische Abbildung aufgebracht werden soll Ein weiterer Gegenstand vorliegender Erfindung sind die beschichteten

Substrate und ein Verfahren zum Aufbringen photographischer Abbildungen auf den Substraten. Die beschichteten Substrate können auch als Aufzeichnungsmaterial für Hologramme (volumen-Phasen-Diagramm) verwendet werden, wobei vorteilhaft ist, dass für diesen Zweck keine Nassentwicklung notwendig ist.

Die Beschichtung der Substrate kann erfolgen, indem man eine flüssige Zusammensetzung, eine Lösung oder Suspension auf das Substrat aufbringt. Flüssige Zusammensetzungen ohne Lösungsmittel sind bevorzugt. Hierbei kann es zweckmässig sein, die erfindungsgemässen Titanocene in Form eines flüssigen Photoinitiatorengemisches, enthaltend andere Photoinitiatoren, z.B. ein Benzilketal, ein 4-Aroyl-1,3-dioxolan, ein Dialkoxyacetophenon, ein $\alpha$-Hydroxy-oder $\alpha$-Aminoacetophenon, ein $\alpha$-Hydroxycycloalkylphenylketon oder Mischungen hiervon einzusetzen. Besonders vorteilhaft sind flüssige Mischungen aus flüssigen bis festen Photoinitiatoren und flüssigen Titanocenen oder flüssigen Photoinitiatoren und sirupösen bis festen Titanocenen. Diese Gemische bieten anwendungstechnische Vorteile und zeichnen sich durch eine hohe Dunkellagerstabilität aus.

Beispiele für Benzilketale sind solche der Formel

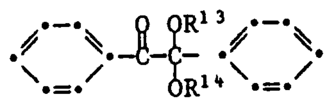

$R^{13}$ = $R^{14}$ = $-CH_3$

$-CH_2CH_3$

$-(CH_2)_2CH_3$

$-(CH_2)_3CH_3$

$-CH_2CH_2CH(CH_3)_2$

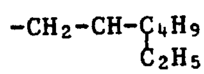

$-(CH_2)_9CH_3$

$-C_{10}H_{21}$-iso

$-C_{12}H_{25}$-n

$-C_9H_{19}$ bis $-C_{11}H_{23}$-Gemisch

$-C_{12}$-$H_{25}$-bis-$C_{15}H_{31}$-Gemisch

$-CH_2CH=CH_2$

$-CH(CH_3)CH=CH_2$

$-CH_2CH_2OC_3H_7$-iso

$-CH_2CH_2OC_4H_9$

$-CH_2CH_2OCH_2CH=CH_2$

$-CH(CH_3)-CH_2OC_4H_9$

$-CH_2COOCH_3$

$-CH_2COOC_4H_9$

$-CH(CH_3)COOCH_3$

$-CH_2CH_2COOC_2H_5$

$-CH(CH_3)CH_2COOCH_3$

$-CH_2CH_2CH(CH_3)OCH_3$

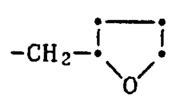

$-(CH_2CH_2O)_2CH_3$

$-(CH_2CH_2O)_2C_2H_5$

$-(CH_2CH_2O)_2C_4H_9$

$-(CH_2CH_2O)_3CH_3$

$-(CH_2CH_2O)_3C_2H_5$

$-(CH_2CH_2O)_3C_{12}H_{25}$
$-(CH_2CH_2O)_5C_{10}H_{21}$
$-(CH_2CH_2O)_8C_9H_{19}$-bis-$C_{11}H_{23}$ (Gemisch)

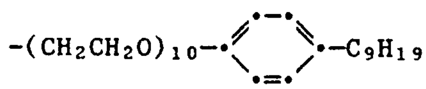

$-CH_2CH_2N(C_2H_5)_2$

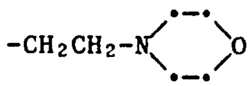

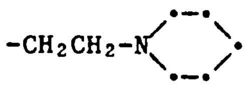

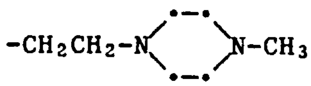

$R^{14}$ = $CH_3$, $R^{13}$ = $C_6H_{13}$
$R^{14}$ = $CH_3$, $R^{13}$ = $C_{10}H_{21}$
$R^{14}$ = $CH_3$, $R^{13}$ = $-(CH_2CH_2O)_3-C_{12}H_{25}$ bis-$C_{15}H_{31}$ (Gemisch)
$R^{14}$ = $CH_3$, $R^{13}$ = $-(CH_2CH_2O)_5-C_9H_{19}$ bis $-C_{11}H_{23}$ (Gemisch)
$R^{14}$ = $CH_3$,

$$R^{13} = -(CH_2CH_2O)_8-C-\overset{O}{\overset{\|}{C}}_{11}H_{23}.$$

Beispiele für 4-Aroyl-1,3-dioxolane sind:
4-Benzoyl-2,2,4-trimethyl-1,3-dioxolan
4-Benzoyl-4-methyl-2,2-tetramethylen-1,3-dioxolan
4-Benzoyl-4-methyl-2,2-pentamethylen-1,3-dioxolan
cis-trans 4-Benzoyl-2,4-dimethyl-2-methoxymethyl-1,3-dioxolan
cis-trans 4-Benzoyl-4-methyl-2-phenyl-1,3-dioxolan
4-(4-Methoxybenzoyl)-2,2,4-trimethyl-1,3-dioxolan
4-(4-Methoxybenzoyl)-4-methyl-2,2-pentamethylen-1,3-dioxolan
4-(4-Methylbenzoyl)-2,2,4-trimethyl-1,3-dioxolan
cis-trans 4-Benzoyl-2-methyl-4-phenyl-1,3-dioxolan
4-Benzoyl-2,2,4,5,5-pentamethyl-1,3-dioxolan
cis-trans 4-Benzoyl-2,2,4,5-tetramethyl-1,3-dioxolan
cis-trans 4-Benzoyl-4-methyl-2-pentyl-1,3-dioxolan
cis-trans 4-Benzoyl-2-benzyl-2,4-dimethyl-1,3-dioxolan
cis-trans 4-Benzoyl-2-(2-furyl)-4-methyl-1,3-dioxolan
cis-trans 4-Benzoyl-5-phenyl-2,2,4-trimethyl-1,3-dioxolan
4-(4-Methoxybenzoyl)-2,2,4,5,5-pentamethyl-1,3-dioxolan.

Beispiele für Dialkoxyacetophenone sind:
$\alpha,\alpha$-Dimethoxyacetophenon
$\alpha,\alpha$-Diethoxyacetophenon
$\alpha,\alpha$-Di-isopropoxyacetophenon
$\alpha,\alpha$-Di-( 2-methoxyethoxy)acetophenon
$\alpha$-Butoxy-$\alpha$-ethoxyacetophenon
$\alpha,\alpha$-Dibutoxy-4-chloracetophenon
$\alpha,\alpha$-Diethoxy-4-fluoracetophenon
$\alpha,\alpha$-Dimethoxy-4-methylacetophenon
$\alpha,\alpha$-Diethoxy-4-methylacetophenon

α,α-Dimethoxypropiophenon
α,α-Diethoxypropiophenon
α,α-Diethoxybutyrophenon
α,α-Dimethoxyisovalerophenon
α,α-Diethoxy-α-cyclohexylacetophenon
α,α-Dipropoxy-4-chlorpropiophenon.

Beispiele für α-Hydroxy- und α-Aminoacetophenone sind:
2-Hydroxy-2-methyl-1-phenyl-propanon-1
2-Hydroxy-2-ethyl-1-phenylhexanon-1
1-(4-Dodecylphenyl)-2-hydroxy-2-methylpropanon-1
1-(2,4-Dimethylphenyl)-2-hydroxy-2-methylpropanon-1
2-Hydroxy-1-(4-methoxyphenyl)-2-ethylpropanon-1
2-Hydroxy-2-methy-1-phenylbutanon-1
2-Dimethylamino-2-methyl-1-phenylpropanon-1
2-Dibutylamino-2-methyl-1-phenylpropanon-1
1-(4-Fluorphenyl)-2-methyl-2-morpholinopentanon-1
2-Methyl-1-(4-methylthiophenyl)-2-morpholinopropanon-1
2-Dimethylamino-1-(4-methoxyphenyl)-2-methylpropanon-1
2-Diethylamino-1-(4-diethylaminophenyl)-2-methylpropanon-1.

Beispiele für α-Hydroxycycloalkylphenylketone sind:
α-Hydroxycyclohexylphenylketon
α-Hydroxycyclopentylphenylketon

Das Photoinitiatorengemisch (b) + (c) kann in Mengen von 0,5-20, vorzugsweise 1 bis 10 Gew.-%, zugegeben werden, bezogen auf die Komponente (a).

Die Wahl des Lösungsmittels und die Konzentration richtet sich hauptsächlich nach der Art der Zusammensetzung und nach dem Beschichtungsverfahren. Die Zusammensetzung wird mittels bekannter Beschichtungsverfahren auf ein Substrat gleichförmig aufgebracht, z.B. durch Tauchen, Rakelbeschichtung, Vorhanggiessverfahren, Elektrophorese, Aufpinseln, Spritzen oder Reverseroll-Beschichtung. Die Auftragsmenge (Schichtdicke) und Art des Substrates (Schichtträger) sind abhängig vom gewünschten Applikationsgebiet. Als Schichtträger für photographische Informationsaufzeichnung dienen z.B. Folien aus Polyester, Celluloseacetat oder mit Kunststoff beschichtete Papiere; für Offsetdruckformen speziell behandeltes Aluminium und für die Herstellung gedruckter Schaltungen kupferkaschierte Laminate. Die Schichtdicken für photographische Materialien und Offsetdruckformen betragen im allgemeinen ca. 0,5 bis ca. 10 µm; für gedruckte Schaltungen im allgemeinen 1 bis ca. 100 µm. Bei Mitverwendung von Lösungsmitteln werden diese nach dem Beschichten entfernt.

Photohärtbare Zusammensetzungen, wie sie für die verschiedenen Zwecke verwendet werden, enthalten meist ausser den photopolymerisierbaren Verbindungen und den Photoinitiatoren eine Reihe sonstiger Zusätze. So ist es vielfach üblich, thermische Inhibitoren zuzusetzen, die vor allem während der Herstellung der Zusammensetzungen durch Mischen der Komponenten vor einer vorzeitigen Polymerisation schützen sollen. Hierzu werden beispielsweise Hydrochinon, Hydrochinonderivate, p-Methoxyphenol, β-Naphthole oder sterisch gehinderte Phenole wie z.B. 2,6-Di(tert-butyl)-p-kresol verwendet. Weiter können geringe Mengen von UV-Absorbern zugesetzt werden, wie z.B. solche vom Benztriazol-, Benzophenon- oder Oxalanilid-Typ. Ebenso lassen sich Lichtschutzmittel vom Typus sterisch gehinderter Amine (HALS) zusetzen.

Zur Erhöhung der Dunkellagerstabilität können Kupferverbindungen, wie Kupfernaphthenat, -stearat, oder -octoat, Phosphorverbindungen, wie Triphenylphosphin, Tributylphosphin, Triethylphosphit, Triphenylphosphit oder Tribenzylphosphit, quaternäre Ammoniumverbindungen, wie Tetramethylammoniumchlorid oder Trimethyl-benzylammoniumchlorid oder Hydroxylaminderivate, wie z.B. N-Diethylhydroxylamin, zugesetzt werden.

Um die inhibierende Wirkung des Luftsauerstoffs auszuschliessen setzt man photohärtbaren Gemischen häufig Paraffin oder ähnliche wachsartige Stoffe zu. Diese schwimmen bei Beginn der Polymerisation wegen mangelnder Löslichkeit im Polymeren aus und bilden eine transparente Oberflächenschicht, die den Zutritt von Luft verhindert.

Weitere übliche Zusätze sind Photosensibilisatoren, welche in bestimmten Wellenlängen absorbieren und die absorbierte Energie an den Initiatoren weitergeben oder selbst als zusätzlicher Initiator fungieren. Beispiele hierfür sind vor allem Thioxanthon-, Anthracen-, Anthrachinon- und Cumarinderivate.

Weitere übliche Zusätze sind Beschleuniger vom Amin-Typ, die vor allem in pigmentierten Zubereitungen von Bedeutung sind, da sie als Kettenüberträger wirken. Beispiele hierfür sind N-Methyldiethanolamin,

Triethylamin, p-Dimethylaminobenzoesäureethylester oder Michler's Keton. Die Wirkung der Amine kann verstärkt werden durch den Zusatz von aromatischen Ketonen vom Benzophenontyp. Weitere übliche Beschleuniger sind 1,3,4-Thiadiazolderivate, wie z.B. 2-Mercapto-5-methylthio-1,3,4-thiadiazol.

Weitere übliche Zusätze sind z.B. Füllstoffe, Pigmente, Farbstoffe, Haft-, Netz- und Verlaufmittel.

Grosse Bedeutung hat die Photohärtung für Druckfarben, da die Trocknungszeit des Bindemittels ein massgeblicher Faktor für die Produktionsgeschwindigkeit graphischer Erzeugnisse ist und in der Grössenordnung von Bruchteilen von Sekunden liegen soll. Insbesondere für den Siebdruck sind UV-härtbare Druckfarben von Bedeutung.

Gut geeignet sind die erfindungsgemässen photohärtbaren Zusammensetzungen auch zur Herstellung von Druckplatten, insbesondere Flexodruckplatten. Hierbei werden z.B. Gemische von löslichen linearen Polyamiden oder von Styrol-Butadien-Kautschuk mit photopolymerisierbaren Monomeren, beispielsweise Acrylamiden oder Acrylaten, und einem Photoinitiator verwendet. Filme und Platten aus diesen Systemen werden über das Negativ (oder Positiv) der Druckvorlage belichtet und die ungehärteten Anteile anschliessend mit einem Lösungsmittel eluiert.

Ein weiteres Einsatzgebiet der Photohärtung ist die Metallbeschichtung, beispielsweise bei der Lackierung von Blechen für Tuben, Dosen oder Flaschenverschlüsse, sowie die Photohärtung von Kunststoffbeschichtungen, beispielsweise von Fussboden- oder Wandbelägen auf PVC-Basis.

Beispiele für die Photohärtung von Papierbeschichtungen sind die farblose Lackierung von Etiketten, Schallplatten-Hüllen oder Buchumschlägen.

Wichtig ist auch die Verwendung der photohärtbaren Zusammensetzungen für Abbildungsverfahren und zur optischen Herstellung von Informationsträgern. Hierbei wird die auf dem Träger aufgebrachte Schicht (nass oder trocken) durch eine Photomaske mit kurzwelligem Licht bestrahlt und die unbelichteten Stellen der Schicht durch Behandlung mit einem Lösungsmittel (= Entwickler) entfernt. Die belichteten Stellen sind vernetztpolymer und dadurch unlöslich und bleiben auf dem Träger stehen. Bei entsprechender Anfärbung entstehen sichtbare Bilder. Ist der Träger eine metallisierte Schicht, so kann das Metall nach dem Belichten und Entwickeln an den unbelichteten Stellen weggeätzt oder durch Galvanisieren verstärkt werden. Auf diese Weise lassen sich gedruckte Schaltungen und Photoresists herstellen.

Zur Belichtung eignen sich Lichtquellen mit hohem Anteil an kurzwelligem Licht. Hierfür stehen heute entsprechende technische Vorrichtungen und verschiedene Lampenarten zur Verfügung. Beispiele sind Kohlelichtbogenlampen, Xenonlichtbogenlampen, Quecksilberdampflampen, Metall-Halogenlampen, Fluoreszenzlampen, Argonlampen oder photographische Flutlichtlampen. Neuerdings werden auch Laserlichtquellen verwendet. Diese haben den Vorteil, dass keine Photomasken notwendig sind; der gesteuerte Laserstrahl schreibt direkt auf die photohärtbare Schicht.

Die erfindungsgemässen Titanocene sind mit den Komponenten der photohärtbaren Zusammensetzungen gut vermischbar bzw. in der Zusammensetzung gut löslich, wodurch eine hohe Lichtempfindlichkeit erzielt werden kann. Sie sind auch leichter zugänglich, da die Lithiumfluorarylpyrrole als Ausgangsprodukte durch einen Lithium/Wasserstoffaustausch erhältlich sind. Eine vorherige Einführung von Halogenen in das Fluorarylpyrrol erübrigt sich daher.

Die nachfolgenden Beispiele erläutern die Erfindung näher.

A) Herstellung der Zwischenprodukte (1-Arylpyrrole)

Beispiele 1 - 7: 1 Mol 2,4-Difluoranilin und 1 mol Diketon (siehe Tabelle 1) werden eine Stunde in 1 l Ethanol am Rückfluss erhitzt. Nach Beendigung der Reaktion (Kontrolle mittels Dünnschichtchromatographie) wird die Mischung im Vakuum vollständig eingedampft und der Rückstand aus heissem Ethanol umkristallisiert. Weitere Angaben sind in Tabelle 1 enthalten.

Beispiel 8: 1 Mol der Verbindung gemäss Beispiel 1 wird mittels $POCl_3$/DMF formyliert [J. Chem. Soc., 1954, 3842]. Das erhaltene Produkt

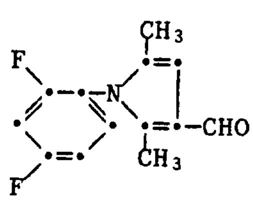

wird mit $(Phenyl)_3PCH(CH_3)_2^{\oplus}I^{\ominus}$/Kalium-butylat in Tetrahydrofuran als Lösungsmittel umgesetzt. Weitere Angaben sind in Tabelle 1 enthalten.

Beispiel 9: 1 Mol der Verbindung gemäss Beispiel 1 wird nach der Vorschrift in Tetrahedron 42 (5), 1299 (1986) mit $CF_3SO_3Si(CH_3)_3$ umgesetzt. Weitere Angaben sind in Tabelle 1 enthalten.

Beispiel 10: 129 g 2,4-Difluoranilin und 136,1 g 2,5-Dimethoxytetrahydrofuran werden in einem Autoklaven während 2 Stunden auf 260 °C erhitzt. Das Reaktionsprodukt wird nach dem Erkalten einer Wasserdampfdestillation unterworfen. Das Destillat wird mit Petrolether ausgeschüttelt, die organische Phase abgetrennt, mit $Na_2SO_4$ getrocknet und im Vakuum eingedampft. Man erhält 167 g eines fast farblosen Oels, das nach kurzem Stehen kristallisiert. Weitere Angaben sind in Tabelle 1 enthalten.

Tabelle 1

| Beispiel | Diketon | Produkt | Isolierung /Reinigung | Ausbeute (%) | Konsistenz | Schmelz-/ Siedepunkt (°C) | Analysen (Zeile 1: berechnet, Zeile 2: gefunden) % C | % H | % F | % N | % O |
|---|---|---|---|---|---|---|---|---|---|---|---|
| 1 | 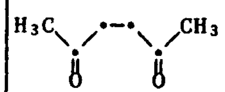 | 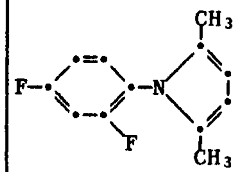 | Umkristallisation Ethanol (- 78°C) | 80 | weisse Nadeln | 68 | 69,56<br>69,54 | 5,35<br>5,35 | 18,34<br>18,12 | 6,76<br>6,78 | |
| 2 | 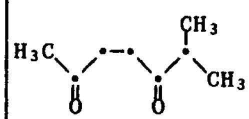 | 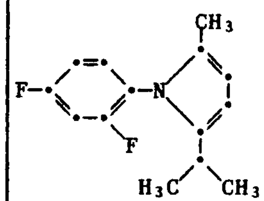 | Chromatographie, $SiO_2$, Hexan, Umkristallisation (- 78°C) | 66 | weisse Kristalle | 27 | 71,47<br>71,57 | 6,43<br>6,48 | 16,15<br>15,99 | 5,95<br>5,97 | |
| 3 | 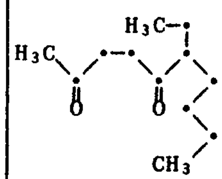 | 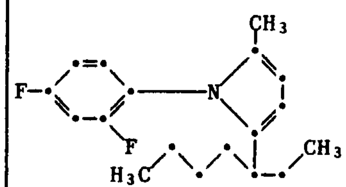 | Umkristallisation Ethanol (- 78°C) | 55 | weisses Pulver | 43 | 74,20<br>74,02 | 7,96<br>8,08 | 13,04<br>13,03 | 4,81<br>4,79 | |
| 4 | 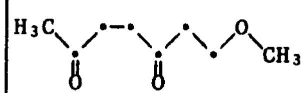 | 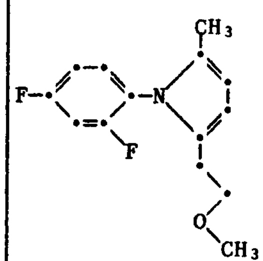 | Chromatographie $SiO_2$/ Hexan mit Ether langsam mischen | 33 | gelbliche ölige Flüssigkeit | | 66,92<br>66,37 | 6,02<br>6,03 | 15,12<br>16,65 | 5,58<br>6,13 | 6,37<br>4,82 |

Tabelle 1: (Fortsetzung)

| Beispiel | Diketon | Produkt | Isolierung /Reinigung | Ausbeute (%) | Konsistenz | Schmelz-/ Siedepunkt (°C) | Analysen (Zeile 1: berechnet, Zeile 2: gefunden) % C | % H | % F | % N | % O |
|---|---|---|---|---|---|---|---|---|---|---|---|
| 5 | 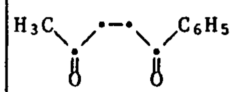 | 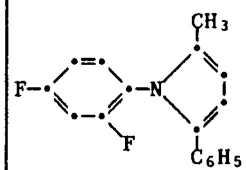 | Umkristallisation Ethanol | 82 | beige Nadeln | 109 | 75,83<br>75,73 | 4,87<br>4,89 | 14,11<br>14,24 | 5,20<br>5,23 | |
| 6 | 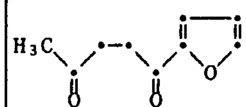 | 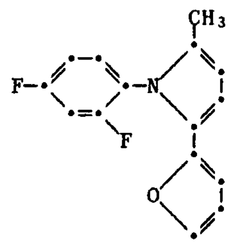 | Chromatographie $SiO_2$/ Hexan | 90 | gelbe Kristalle | 44 | 69.49<br>69.66 | 4.28<br>4.43 | 14.66<br>14.52 | 5.40<br>5.37 | 6.17<br>6.02 |
| 7 | 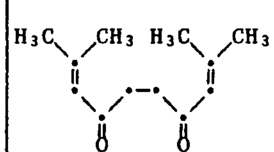 | 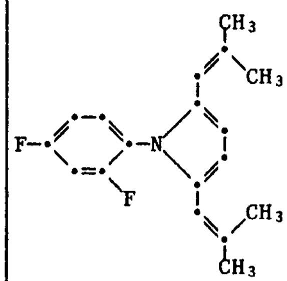 | Umkristallisation Ethanol | 39 | gelbliche Kristalle | 104 | 75.24<br>75.10 | 6.67<br>6.68 | 13.22<br>13.27 | 4.88<br>4.99 | -<br>- |

Tabelle 1: (Fortsetzung)

| Bsp. | Edukt | Produkt | Isolierung /Reinigung | Ausbeute (%) | Konsistenz | Schmelz-/ Siedepunkt (°C) | Analysen (Zeile 1: berechnet, Zeile 2: gefunden) % C | % H | % F | % N | % O |
|---|---|---|---|---|---|---|---|---|---|---|---|
| 8 | 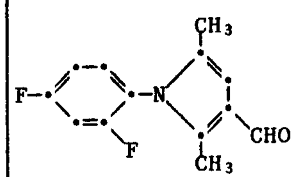 $Ph_3PCH(CH_3)_2^+I^-$/KO-t-Butyl | 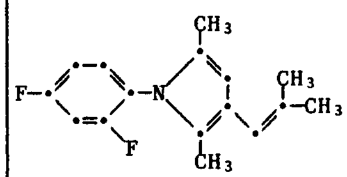 | Chromatographie $SiO_2$/ Hexan: Ether (1:1) | 82 | gelbliche Flüssigkeit | | 73.54<br>73.34 | 6.56<br>6.69 | 14.54<br>14.17 | 5.36<br>5.37 | |
| 9 | 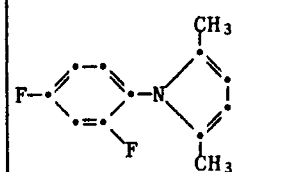 $CF_3SO_3$ $Si(CH_3)_3$ | 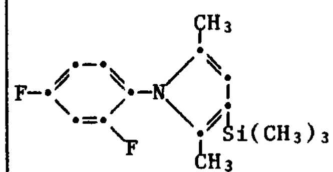 | Kugelrohrdestillation | 65 | hellgelbe Flüssigkeit | 170<br>$2x10^{-1}$ mbar | 64.48<br>64.36 | 6.86<br>6.91 | 13.60<br>13.55 | 5.01<br>5.08 | Si<br>10.05<br>10.04 |
| 10 | 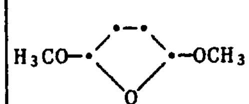 2,4-Difluoranilin | 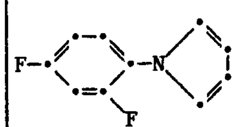 | Wasserdampfdestillation | 93 | farblose Kristalle | 42 | 67.04<br>67.11 | 3.94<br>3.99 | 21.21<br>21.37 | 7.82<br>7.92 | -<br>- |

Beispiel 11: Zu 0,1 mol 1-(2,4-Difluorphenyl)-2,5-dimethylpyrrol (Produkt von Beispiel 1) wird eine Lösung, bestehend aus 18 g 40 % Dimethylamin in Wasser (= 0,16 mol Dimethylamin), 13,7 g 35 %ige Formalinlösung (= 0,16 mol Formaldehyd) und 15 ml Eisessig zugetropft. Die entstehende Suspension wird bei Raumtemperatur gerührt, bis gemäss chromatographischer Analyse das Edukt umgesetzt ist. Mit verdünnter Natronlauge wird basisch gestellt, das Produkt in Ether aufgenommen und mit Wasser gewaschen. Das nach Einengen der Lösung anfallende Rohprodukt kann als solches für die nächste Stufe

verwendet oder durch eine Kurzwegdestillation weiter gereinigt werden. Weitere Angaben sind in Tabelle 2 enthalten.

Beispiele 12 - 21: Die Verbindungen wurden nach dem für Beispiel 11 gegebenen Verfahren hergestellt. Die verwendeten Edukte, Molequivalente und Reaktionstemperaturen sind in Tabelle 2 angegeben.

Tabelle 2:

| Beispiel | Pyrrol | Amin (Molequivalente Amin und $CH_2O$) Reaktionstemperatur | Produkt | Ausbeute | Analysen (Zeile 1: berechnet, Zeile 2: gefunden) % C | % H | % N |
|---|---|---|---|---|---|---|---|
| 11 | Verbindung aus Bsp. 1 | $HN(CH_3)_2$ (1,6) RT | 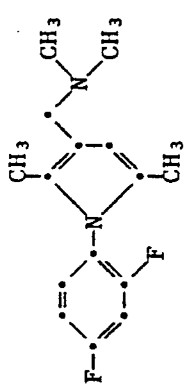 | 84 % | 68,16<br>67,83 | 6,86<br>6,91 | 10,60<br>10,55 |
| 12 | Verbindung aus Bsp. 1 | $HN(CH_3)_2$ (3,2) 50°C | 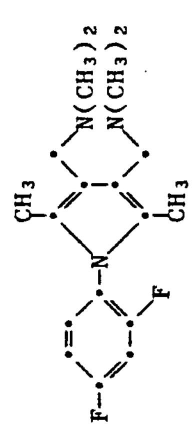 | 88 % | 67,26<br>66,99 | 7,84<br>7,91 | 13,07<br>13,12 |
| 13 | Verbindung aus Bsp. 1 | 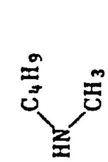 (1,6) RT | 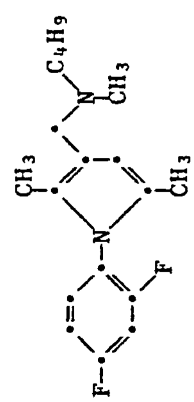 | 99 % | 70,56<br>69,48 | 7,90<br>8,32 | 9,14<br>9,54 |
| 14 | Verbindung aus Bsp. 1 | $HN(C_4H_9)_2$ (1,6) RT | 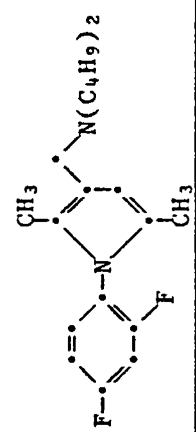 | 53 % | 72,38<br>72,43 | 8,68<br>8,46 | 8,04<br>7,78 |

Tabelle 2: (Fortsetzung)

| Beispiel | Pyrrol | Amin (Molequivalente Amin und $CH_2O$) Reaktionstemperatur | Produkt | Ausbeute | Analysen (Zeile 1: berechnet, Zeile 2: gefunden) % C | % H | % N |
|---|---|---|---|---|---|---|---|
| 15 | Verbindung aus Bsp. 1 | $HN(CH_2CH_2OCH_3)_2$ (1,6) RT | 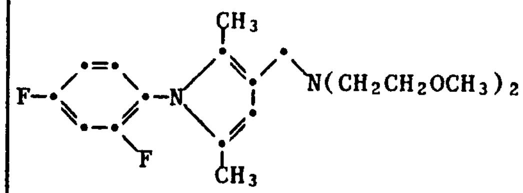 | 69 % | 64,75<br>64,34 | 7,44<br>7,52 | 7,95<br>7,86 |
| 16 | Verbindung aus Bsp. 1 | 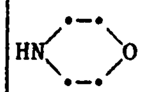 (1,6) RT | 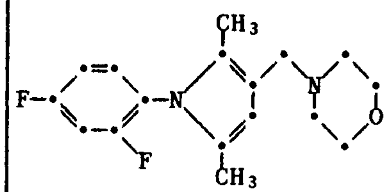 | 45 % | 66,65<br>66,44 | 6,58<br>6,51 | 9,14<br>8,96 |
| 17 | Verbindung aus Bsp. 10 | $HN(CH_3)_2$ (3,2) 50°C | 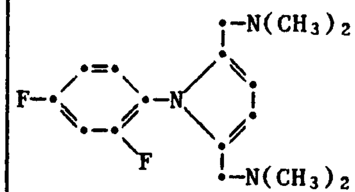 | 76 % | 65,51<br>65,51 | 7,22<br>7,15 | 14,32<br>14,07 |
| 18 | Verbindung aus Bsp. 10 | 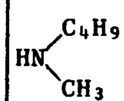 (3,2) 50°C | 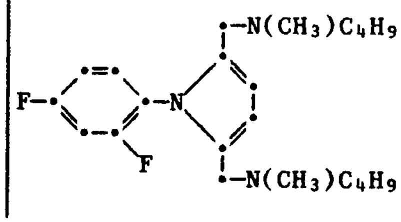 | 93 % | 69,99<br>69,61 | 8,81<br>8,94 | 11,13<br>11,01 |

Tabelle 2: (Fortsetzung)

| Beispiel | Pyrrol | Amin (Molequivalente Amin und $CH_2O$) Reaktionstemperatur | Produkt | Ausbeute | Analysen (Zeile 1: berechnet, Zeile 2: gefunden) % C | % H | % N |
|---|---|---|---|---|---|---|---|
| 19 | Verbindung aus Bsp. 10 | $HN(C_4H_9)_2$ (3,2) 50°C | 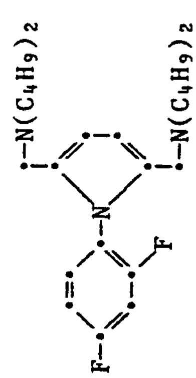 | 68 % | 72,84<br>72,60 | 9,82<br>9,96 | 9,10<br>9,32 |
| 20 | Verbindung aus Bsp. 10 | $HN(CH_2CH_2OCH_3)_2$ (3,2) 50°C | 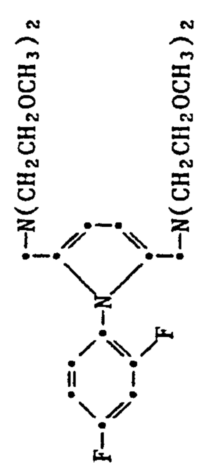 | 81 % | 61,26<br>61,35 | 8,14<br>7,92 | 8,93<br>8,76 |
| 21 | Verbindung aus Bsp. 10 | 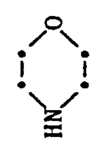 (3,2) 50 °C | 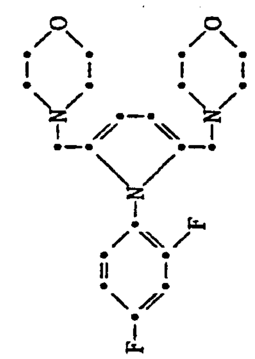 | 95 % | 63,65<br>63,38 | 6,68<br>7,02 | 11,13<br>11,36 |

Beispiele 22 und 23: 0,2 Mol des in Tabelle 3 angegebenen Pyrrolaldehydes (hergestellt durch Formylierung des entsprechenden Pyrrols mit $POCl_3$/DMF nach J. Chem. Soc. 1954, 3842) werden in 400 ml trockenem Diethylether mit 0,05 mol $LiAlH_4$ versetzt und die Suspension zum Rückfluss erwärmt, bis im Dünnschicht-Chromatogramm kein Edukt mehr nachgewiesen werden kann. Nach Hydrolyse mit wässriger $MgSO_4$-Lösung wird die Etherphase getrocknet und eingedampft, wobei der entsprechende Alkohol (Hydroxymethylpyrrol) hinterbleibt. Dieser wird mit einer Suspension von 0,2 mol NaH in 100 ml trockenem Diethylether gerührt, bis im DünnschichtChromatogramm kein Alkohol mehr nachgewiesen werden kann. Danach werden 0,3 mol Methyliodid zugegeben und über Nacht gerührt. Nach Waschen mit Wasser wird die Etherlösung getrocknet und eingedampft. Das rohe Methoxymethylpyrrol wird durch Säulenchromatographie (Kieselgel/Hexan/Ethylacetat) gereinigt. Weitere Angaben siehe Tabelle 3

Beispiel 24: 0,1 Mol des Aminomethylpyrrols aus Beispiel 11 wird in 20 ml Ethanol gelöst und unter Kühlen mit 0,1 mol Methyliodid versetzt. Nach 30 Min. wird das ausgefallene Salz abfiltriert, mit Ethanol gewaschen und zu einer Lösung von 0,14 mol $C_2H_5ONa$ in Ethanol gegeben. Die Suspension wird 4 Tage zum Rückfluss erwärmt und dann auf Wasser gegossen. Das Produkt wird mit Diethylether extrahiert und durch Chromatographie an Kieselgel (Hexan/Ethylacetat 9:1) gereinigt. Weiter Angaben in Tabelle 3.

**Tabelle 3:**

| Bsp. | Edukt | Produkt | Ausbeuten (1.Stufe/ 2. Stufe) | Analysen (Zeile 1: berechnet, Zeile 2: gefunden) % C | % H | % N |
|---|---|---|---|---|---|---|
| 22 | 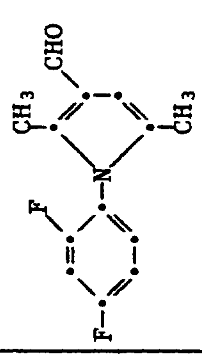 | 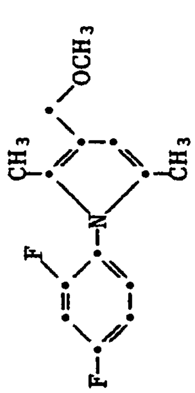 | 78 %<br>56 % | 66,92<br>67,02 | 6,02<br>5,99 | 5,57<br>5,62 |
| 23 | 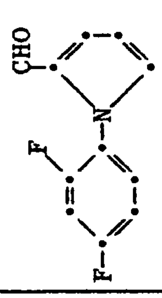 | 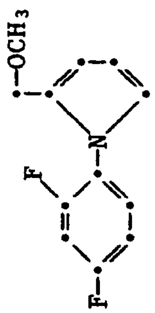 | 88 %<br>37 % | 64,57<br>64,51 | 4,97<br>5,19 | 6,27<br>6,07 |
| 24 | 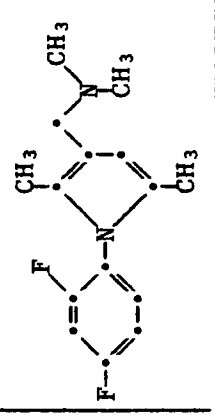 | 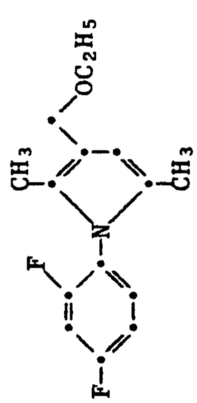 | 11 % | 67,91<br>67,82 | 6,46<br>6,47 | 5,28<br>5,13 |

B) Herstellung der Titanocene

Beispiele 25 - 35: Unter Argon wird die jeweils in Tabelle 4 angegebene Menge Fluoraromat im jeweiligen Lösungsmittel gelöst, bei -70 °C tropfenweise mit der entsprechenden Menge 1,6 molarer Butyllithium-Hexan-Lösung versetzt und noch eine Stunde bei -70 °C gerührt. Anschliessend wird die

angegebene Menge Titanocendichlorid in einer Portion zugegeben, die Reaktionsmischung langsam auf Raumtemperatur erwärmt und noch drei Stunden bei Raumtemperatur gerührt. Danach wird im Vakuum vollständig eingedampft, der Rückstand mit Dichlormethan oder Chloroform extrahiert und die Extrakte filtriert. Das Filtrat wird erneut eingedampft und der verbleibende Rückstand wie in Tabelle 5 angegeben gereinigt.

Die Produkte haben gelb-orange bis rot-orange Farbe. Die Produkte sind kristallin. Alle Produkte sind unter Lichtausschuss stabil und nicht luftempfindlich.

In den folgenden Tabellen bedeuten Cp Cyclopentadienyl und Me Methyl. Die chromatographische Reinigung geschieht an Säulen aus Aluminiumoxid (Woelm) neutral (= $Al_2O3$) oder aus Kieselgel 60 (Merck) (= $SiO_2$).

Beispiel 36: 44,7 g N-2,4-Difluorphenyl-pyrrol werden in 750 ml absolutem Diethylether gelöst und auf -75 °C abgekühlt. Unter $N_2$ werden 170 ml einer 15 %igen Hexanlösung von Butyllithium zugetropft. Nach 1 stündigem Rühren bei -75 °C werden 31,1 g Titanocendichlorid zugegeben. Nach dem Entfernen der Kühlung und dem Erreichen der Raumtemperatur wird die orange Suspension in 2 l Ethylacetat aufgenommen, dieses 3x mit $H_2O$ gewaschen, mit $Na_2SO_4$ getrocknet und am Rotationsverdampfer eingedampft. Man erhält 73,5 g eines orangen Harzes.

Das orange Harz wird über Nacht mit 250 ml Ethanol verrührt, wobei Kristallisation eintritt. Nach dem Filtrieren und dem Waschen mit etwas Ethanol erhält man 53.7 g helloranger Kristalle vom Fp. 165 - 166 °C, Ausbeute 80,4 %. Zur Analyse kann das Produkt aus Aethanol umkristallisiert werden.

Beispiele 37 - 42: Es wurde wie in den Beispielen 25 - 35 vorgegangen. Weitere Angaben sind in den Tabellen 4 und 5 angeführt.

Tabelle 4

| Beispiel | Titanverbindung/Menge | Fluoraromat gemäss Beispiel/Menge | Butyllithium 1.6 m in Hexan | Lösungsmittel |
|---|---|---|---|---|
| 25 | $cp_2TiCl_2$/6,5 g | 1: 10,4 g | 31,25 ml | THF 250 ml |
| 26 | $(Me_3Sicp)_2$ $TiCl_2$/10,2 g | 1: 10,4 g | 31,25 ml | THF 250 ml |
| 27 | (Me cp)$_2$ $TiCl_2$/7,2 g | 1: 10,4 g | 31,25 ml | THF 250 ml |
| 28 | $cp_2$ $TiCl_2$/4,8 g | 2: 8,7 g | 23.1 ml | THF 250 ml |
| 29 | $cp_2$ $TiCl_2$/2,5 g | 3: 5,8 g | 12,5 ml | THF 140 ml |
| 30 | $cp_2$ $TiCl_2$/2,0 g | 4: 4,0 g | 10 ml | THF 110 ml |
| 31 | $cp_2$ $TiCl_2$/7,7 g | 5: 16,2 g | 37,5 ml | THF 400 ml |
| 32 | $cp_2$ $TiCl_2$/2,2 g | 6: 4,5 g | 10,9 ml | THF 120 ml |
| 33 | $cp_2$ $TiCl_2$/4,2 g | 7: 9,2 g | 20,05 ml | THF 200 ml |
| 34 | $cp_2$ $TiCl_2$/2,4 g | 8: 5,0 g | 12,0 ml | THF 100 ml |
| 35 | $cp_2$ $TiCl_2$/6,0 g | 9: 13,0 g | 29,1 ml | THF 300 ml |
| 37 | $cp_2$ $TiCl_2$/1,9 g | 11: 4,0 g | 20,0 ml | THF 40 ml |
| 38 | $cp_2$ $TiCl_2$/3,7 g | 12: 10,4 g | 20,3 ml | THF 220 ml |
| 39 | $cp_2$ $TiCl_2$/3,7 g | 15: 11,4 g | 20,3 ml | THF 220 ml |
| 40 | $cp_2$ $TiCl_2$/1,9 g | 16: 4,6 g | 10,0 ml | THF 40 ml |
| 41 | $cp_2$ $TiCl_2$/3,7 g | 17: 9,5 g | 20,3 ml | THF 220 ml |
| 42 | $cp_2$ $TiCl_2$/1,9 g | 21: 5,1 g | 10,0 ml | THF 40 ml |

**Tabelle 5**

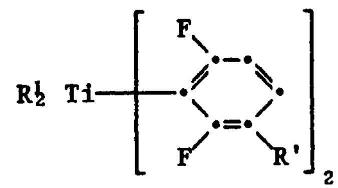

| Bei-spiel | $R^1$ | R' | Reinigung | Schmelzpunkt (°C) |
|---|---|---|---|---|
| 25 | Cp | 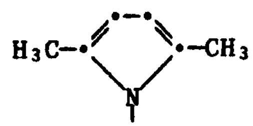 | Umkristallisation Diethylether/Hexan | 205 (Zersetzung) |
| 26 | $Me_3SiCp$ | 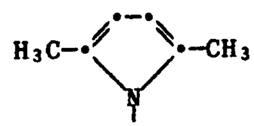 | Waschen mit Pentan | 113 |
| 27 | MeCp | 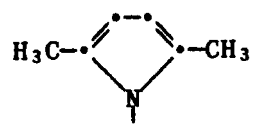 | Waschen mit Pentan | 106 |
| 28 | Cp | 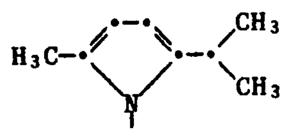 | Chromatographie: Hexan; Hexan/Diethylether | 197 |
| 29 | Cp | 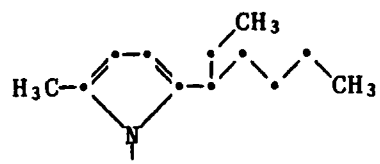 | Chromatographie wie Beispiel 28 | 74 |

Tabelle 5 (Fortsetzung)

| Beispiel | $R^1$ | R' | Reinigung | Schmelzpunkt (°C) |
|---|---|---|---|---|
| 30 | Cp | 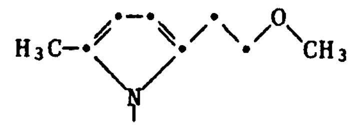 | Chromatographie: Hexan/Ether | 72 |
| 31 | Cp | 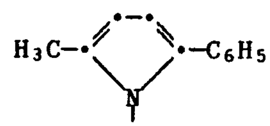 | Umkristallisation Diethylether/ Pentan | 200 |
| 32 | Cp | 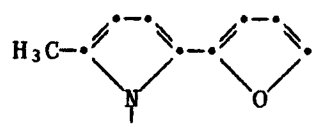 | Umkristallisation 1. Hexan 2. Diethylether | 203 |
| 33 | Cp | 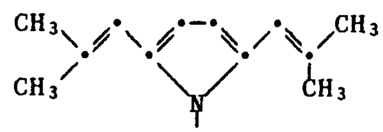 | Chromatographie wie Beispiel 28, Umkristallisation aus Diäthylether/ Pentan | Zersetzung > 100 |
| 34 | Cp | 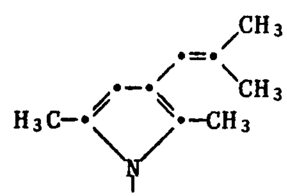 | Umkristallisation Diethylether | Zersetzung > 100 |

Tabelle 5 (Fortsetzung)

| Beispiel | $R^1$ | R' | Reinigung | Schmelzpunkt (°C) |
|---|---|---|---|---|
| 35 | Cp | 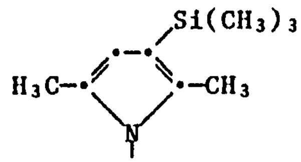 | Waschen mit Hexan | 130 |
| 36 | Cp | 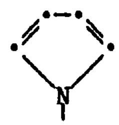 | Umkristallisation Ethanol | 160 |
| 37 | Cp | 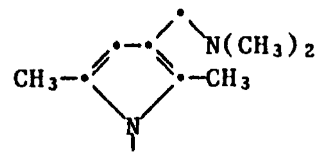 | Umkristallisation aus $CH_2Cl_2$/Hexan | 75-80 |
| 38 | Cp | 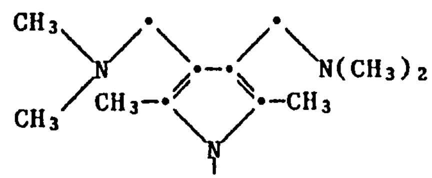 | Extraktion mit $H_2O$; Umkristallisation aus Hexan | 108 |
| 39 | Cp | 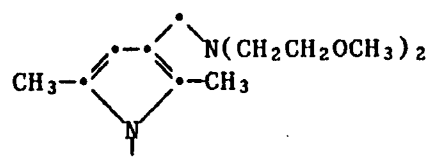 | Waschen mit Hexan Umkristallisation aus $CH_2Cl_2$/Hexan | ab 70 |

Tabelle 5 (Fortsetzung)

| Bei-spiel | $R^1$ | R' | Reinigung | Schmelzpunkt (°C) |
|---|---|---|---|---|
| 40 | Cp | 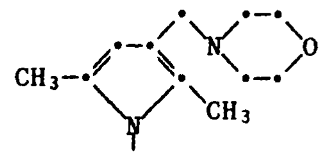 | Waschen mit Hexan; Umkristallisation aus $CH_2Cl_2$/Hexan | 197-200 |
| 41 | Cp | 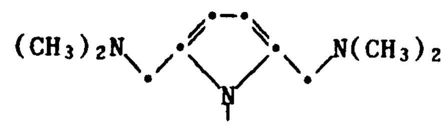 | Umkristallisation aus $CH_2Cl_2$/Hexan | 175 |
| 42 | Cp | 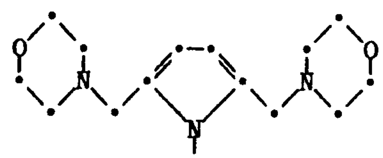 | Chromatographie Umkristallisation aus $CH_2Cl_2$/Hexan | 100-105 |
| 43 | MeCp | 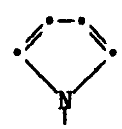 | Chromatographie Ethylacetat/Hexan | 85° |

c) Anwendungsbeispiele

Beispiel 43: Photohärtung eines Acrylat-Gemisches. Es wird eine photohärtbare Zusammensetzung hergestellt durch Mischen der folgenden Komponenten:

| | Feststoffgehalt |
|---|---|
| 150,30 g Scripset 540[1] (30 %-ige Lsg. in Aceton) | 45,1 g |
| 48,30 g Trimethylolpropantriacrylat | 48,3 g |
| 6,60 g Polyethylenglykoldiacrylat | 6,6 g |
| 0,08 g Kristallviolett | |
| 205,28 g | 100,0 g |

[1] Polystyrol-Maleinsäureanhydrid-Copolymer (Monsanto)

Portionen dieser Zusammensetzung werden mit den in der folgenden Tabelle angegebenen Menge an Photoinitiator oder Initiatorgemischen vermischt. Bei den Initiatorgemischen handelt es sich um Lösungen eines Titanocens in einem flüssigen Initiator aus einer 1:1-Mischung von α-Hydroxcyclohexylphenylketon und Benzophenon. Alle Operationen werden unter Rotlicht oder Gelblicht ausgeführt.

Die mit Initiator versetzten Proben werden in einer Stärke von 150 µm auf 200 µm Aluminiumfolie (10 x 15 cm) aufgetragen. Das Lösungsmittel wird durch Erwärmung auf 60 °C während 15 Minuten im Umluftofen entfernt. Auf die flüssige Schicht wird eine 76 µm dicke Polyesterfolie gelegt und auf diese ein standardisiertes Testnegativ mit 21 Stufen verschiedener optischer Dichte (Stauffer-Keil) gelegt. Darüber wird eine zweite Polyesterfolie gelegt und das so erhaltene Laminat auf einer Metallplatte fixiert. Die Probe wird dann mit einer 5 KW-Metallhalogenid-Lampe im Abstand von 30 cm belichtet und zwar in einer ersten Testreihe 20 Sekunden und einer zweiten Testreihe 40 Sekunden. Nach der Belichtung werden die Folien und die Maske entfernt, die belichtete Schicht in einem Ultraschallbad 120 Sekunden mit Entwickler A entwickelt und anschliessend bei 60 °C 15 Minuten im Umluftofen getrocknet. Die Empfindlichkeit des verwendeten Initiatorsystems wird durch die Angabe der letzten klebefrei abgebildeten Keilstufe charakterisiert. Je höher die Zahl der Stufen ist, desto empfindlicher ist das System. Eine Erhöhung um zwei Stufen bedeutet dabei etwa eine Verdopplung der Härtungsgeschwindigkeit. Die Ergebnisse sind in Tabelle 6 angegeben. Entwickler A enthält 15 g Natriummetasilikat•$9H_2O$; 0,16 g KOH; 3 g Polyethylenglykol 6000; 0,5 g Lävulinsäure und 1000 g deionisiertes Wasser.

Tabelle 6

| Menge Photoinitiator *) | | Zahl der abgebildeten Stufen | |
|---|---|---|---|
| Titanocen von Beispiel | Initiator | nach 20 s | nach 40 s Belichtung |
| 0,3 % von 14 | - | 17 | 19 |
| 0,3 % von 14 | 1,7 % | 18 | 20 |
| 0,3 % von 15 | - | 14 | 16 |
| 0,3 % von 15 | 1,7 % | 15 | 18 |
| 0,3 % von 17 | - | 14 | 16 |
| 0,3 % von 17 | 1,7 % | 16 | 18 |
| 0,3 % von 20 | - | 16 | 18 |
| 0,3 % von 20 | 1,7 % | 17 | 19 |
| 0,3 % von 16 | - | 15 | 17 |
| 0,3 % von 18 | - | 11 | 13 |
| 0,3 % von 19 | - | 13 | 15 |
| 0,3 % von 21 | - | 14 | 16 |
| 0,3 % von 25 | - | 16 | 20 |
| 0,3 % von 26 | - | 13 | 16 |
| 0,3 % von 36 | - | 15 | 18 |
| 0,3 % von 43 | - | 12 | 14 |
| 0,2 % von 11 | - | 15 | 17 |
| 0,2 % von 12 | - | 11 | 13 |
| 0,2 % von 13 | - | 10 | 12 |

*) bezogen auf die photohärtbare Zusammensetzung

Beispiel 44: Photohärtung eines Acrylat-Gemisches

Es wird eine photohärtbare Zusammensetzung hergestellt durch Mischen der folgenden Komponenten:
37,64 g Sartomer SR 444 (Pentaerythritol-triacrylat) (Sartomer Company, Westchester)
10,76 g Cymel 301 Hexamethoxymethylmelamin (Cyanamid)
47,30 g Carboset 525 (Thermoplastisches Acrylat mit Carboxylgruppen/B.F. Goodrich)
4,30 g Polyvinylpyrrolidon PVP (GAF)
100,00 g der obigen Mischung
0,50 g Irgalitgrün GLN
319,00 g Methylenchlorid
30,00 g Methanol
450,00 g
Portionen dieser Zusammensetzung werden mit den in der folgenden Tabelle angegebenen Mengen an Titanocen vermischt. Alle Operationen werden unter Rotlicht oder Gelblicht ausgeführt.

Die mit Initiator versetzten Proben werden in einer Stärke von 200 µm auf 200 µm Aluminiumfolie (10 x 15 cm) aufgetragen. Das Lösungsmittel wird durch Erwärmung auf 60 °C während 15 Minuten im Umluftofen entfernt. Auf die flüssige Schicht wird eine 76 µm dicke Polyesterfolie gelegt und auf diese ein standardisiertes Testnegativ mit 21 Stufen verschiedener optische Dichte (Stauffer-Keil) gelegt. Darüber wird eine zweite Polyesterfolie gelegt und das so erhaltene Laminat auf einer Metallplatte fixiert. Die Probe wird dann mit einer 5 KW-Metallhalogenid-Lampe im Abstand von 30 cm belichtet und zwar in einer ersten Testreihe 20 Sekunden und in einer zweiten Testreihe 40 Sekunden. Nach der Belichtung werden die Folien und die Maske entfernt, die belichtete Schicht in einem Ultraschallbad 240 Sekunden mit Entwickler A entwickelt und anschliessend bei 60 °C 15 Min. im Umluftofen getrocknet. Die Empfindlichkeit des verwendeten Initiatorsystems wird durch die Angabe der letzten klebefrei abgebildeten Keilstufe charakterisiert. Je höher die Zahl der Stufen ist, desto empfindlicher ist das System. Eine Erhöhung um zwei Stufen bedeutet dabei etwa eine Verdopplung der Härtungsgeschwindigkeit. Die Ergebnisse sind in Tabelle 7 angegeben.

Tabelle 7

| Menge von Titanocen*) von Beispiel | Zahl der abgebildeten Stufen | |
|---|---|---|
| | nach 20 s | nach 40 s Belichtung |
| 0,3 % von 14 | 13 | 15 |
| 0,3 % von 15 | 12 | 15 |
| 0,3 % von 16 | 11 | 14 |
| 0,3 % von 17 | 12 | 14 |
| 0,3 % von 18 | 10 | 13 |
| 0,3 % von 19 | 10 | 13 |
| 0,3 % von 20 | 11 | 13 |
| 0,3 % von 21 | 12 | 14 |
| 0,3 % von 25 | 13 | 16 |
| 0,3 % von 36 | 15 | 17 |
| 0,3 % von 39 | 8 | 10 |
| 0,3 % von 42 | 7 | 10 |
| 0,3 % von 43 | 13 | 15 |

*) bezogen auf die lösungsmittelfreie photohärtbare Zusammensetzung

## Patentansprüche

**1.** Titanocene der Formel I

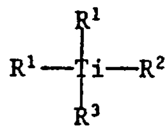

(I),

worin beide $R^1$ unabhängig voneinander gegebenenfalls ein- oder mehrfach durch $C_1$-$C_{18}$-Alkyl oder -Alkoxy, $C_2$-$C_{18}$-Alkenyl, $C_5$-$C_8$-Cycloalkyl, $C_6$-$C_{16}$-Aryl, $C_7$-$C_{16}$-Aralkyl, $SiR^4{}_3$, $GeR^4{}_3$, Cyano oder Halogen substituiertes Cyclopentadienyl$^{\ominus}$, Indenyl$^{\ominus}$, 4,5,6,7-Tetrahydroindenyl$^{\ominus}$ bedeuten oder beide $R^1$ zusammen für einen gegebenenfalls wie zuvor substituierten Rest der Formel II

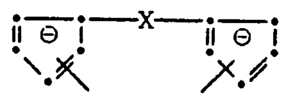

(II)

stehen, worin X $-(CH_2)_n-$ mit n = 1, 2 oder 3, gegebenenfalls durch Phenyl substituiertes Alkyliden mit 2 bis 12 C-Atomen, Cycloalkyliden mit 5 bis 7 Ringkohlenstoffatomen, $SiR^4{}_2$, $SiR^4{}_2$-O-$SiR^4{}_2$ , $GeR^4{}_2$ oder $SnR^4{}_2$ ist, und $R^4$ $C_1$-$C_{12}$-Alkyl, $C_5$-$C_{12}$-Cycloalkyl, $C_6$-$C_{16}$-Aryl oder $C_7$-$C_{16}$-Aralkyl bedeutet,
$R^2$ einen 6-gliedrigen carbocyclischen oder 5- oder 6-gliedrigen heterocyclischen aromatischen Ring bedeutet, der in mindestens einer der beiden ortho-Stellungen zur Titankohlenstoffbindung mit Fluoratomen substituiert ist und wobei der aromatische Ring weitere Substituenten enthalten kann, $R^3$ unabhängig die Bedeutung von $R^2$ hat oder $R^2$ und $R^3$ zusammen einen Rest der Formel III bedeuten,

-Q-Y-Q- (III)

in dem Q für einen carbocyclischen aromatischen Ring steht, wobei die beiden Bindungen jeweils in Orthostellung zur Y-Gruppe stehen und die zweiten Orthostellungen zur Titankohlenstoffbindung jeweils durch ein Fluoratom substituiert sind und wobei Q weitere Substituenten enthalten kann, und Y $CH_2$, Alkyliden mit 2 bis 12 C-Atomen, Cycloalkyliden mit 5 bis 7 Ringkohlenstoffatomen, $NR^4$, O, S, SO, $SO^2$, CO, $SiR^4{}_2$, $GeR^4{}_2$ oder $SnR^4{}_2$ bedeutet und $R^4$ die zuvor angegebene Bedeutung hat, wobei die Titanocene dadurch gekennzeichnet sind, dass $R^2$ und $R^3$ durch einen Rest der Formel IV substituiert sind,

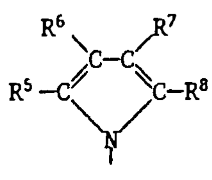

(IV)

worin $R^5$, $R^6$, $R^7$ und $R^8$ unabhängig voneinander ein Wasserstoffatom, unsubstituiertes oder mit $C_2$-$C_8$-Dialkylamino oder -aminomethyl, Bis[2-($C_1$-$C_4$-alkoxy)ethyl]amino oder -aminomethyl, Morpholino, Morpholinylmethyl, Piperidino, Piperidinylmethyl, N-Methylpiperazino, N-Methylpiperazinylmethyl, Pyrrolidino, Pyrrolidinylmethyl, quaternärem $C_3$-$C_{10}$-Trialkylammonium oder -ammoniummethyl, $C_1$-$C_{12}$-Alkoxy,

$$-(OCH_2CH_2)_l-O-C_1-C_{16}\text{-Alkyl},$$

worin l eine Zahl von 1 bis 20 ist, 1,3-Dioxolan-2-yl, 4-Methyl-1,3-dioxolan-2-yl, $-OCH_2CH_2O-$, $C_1$-$C_{12}$-Alkoxycarbonyl, $C_2$-$C_{12}$-Alkanoyloxy, $C_2$-$C_{12}$-Alkanoyl, $C_1$-$C_{12}$-Alkylthio, Halogen, Cyano oder $SiR^4{}_3$, worin $R^4$ die zuvor angegebene Bedeutung hat, substituiertes lineares oder verzweigtes $C_1$-$C_{18}$-Alkyl, $C_2$-$C_5$-Alkenyl, $C_7$-$C_9$-Aralkyl oder -Alkaryl, $C_8$-$C_{10}$-Alkaralkyl, $C_6$-$C_{10}$-Aryl, 2-Furyl, $C_5$-$C_8$-Cycloalkyl,

$C_5$-$C_8$-Cycloalkenyl, $C_2$-$C_{12}$-Alkanoyl oder $C_2$-$C_{12}$-Alkoxycarbonyl bedeuten; oder -CHO, -$SiR^4_3$ oder -$GeR^4_3$ darstellen, worin $R^4$ die zuvor angegebene Bedeutung hat; oder $R^5$ und $R^6$ und/oder $R^7$ und $R^8$ oder $R^6$ und $R^7$ jeweils zusammen $-(CH_2)_3-$, $-(CH_2)_4-$, -CH=CH-CH=CH-, -CH=CH-C($R^{11}$)=CR-, $-CH_2OCH_2-$oder $-CH_2N(C_1$-$C_4$-Alkyl)$CH_2-$ sind, worin $R^{11}$ Hydroxyl, $C_1$-$C_4$-Alkoxy oder $C_2$-$C_4$-Alkanoyloxy bedeutet.

2. Titanocene gemäss Anspruch 1, dadurch gekennzeichnet, dass $R^1$ Cyclopentadienyl$^\ominus$ oder Methylcyclopentadienyl$^\ominus$ ist.

3. Titanocene gemäss Anspruch 1, dadurch gekennzeichnet, dass $R^2$ und $R^3$ die gleiche Bedeutung haben.

4. Titanocene gemäss Anspruch 1, dadurch gekennzeichnet, dass der Rest $R^2$ in beiden Orthostellungen zur Titankohlenstoffbindung mit Fluor substituiert ist.

5. Titanocene gemäss Anspruch 1, dadurch gekennzeichnet, dass $R^2$ und $R^3$ für 2,6-Difluorphen-1-yl stehen, an das ein Rest der Formel IV gebunden ist, und das weitere 1 oder 2 gleiche oder verschiedene Substituenten enthalten kann.

6. Titanocene gemäss Anspruch 5, dadurch gekennzeichnet, dass in Formel I beide $R^1$ Cyclopentadienyl$^\ominus$ oder durch $C_1$-$C_4$-Alkyl substituiertes Cyclopentadienyl$^\ominus$ und $R^2$ und $R^3$ Reste der Formel V

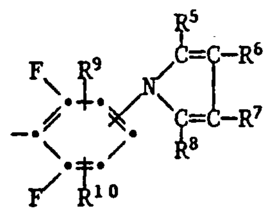

(V)

bedeuten, worin $R^5$, $R^6$, $R^7$ und $R^8$ die in Anspruch 1 angegebene Bedeutung haben, und $R^9$ und $R^{10}$ unabhängig voneinander für H oder F stehen.

7. Titanocene gemäss Anspruch 6, dadurch gekennzeichnet, dass in Formel V die Pyrrolgruppe in Orthostellung zu einem F-Atom gebunden ist.

8. Titanocene gemäss Anspruch 6, dadurch gekennzeichnet, dass $R^9$ und $R^{10}$ für H stehen.

9. Titanocene gemäss Anspruch 1, dadurch gekennzeichnet, das $R^5$, $R^6$, $R^7$ und $R^8$ unabhängig voneinander ein Wasserstoffatom oder unsubstituiertes oder substituiertes $C_1$-$C_{12}$-Alkyl, $C_2$-$C_5$-Alkenyl, $C_7$- oder $C_8$-Aralkyl oder -Alkaryl, Phenyl, 2-Fury, $C_5$- oder $C_6$-Cycloalkyl, $C_5$- oder $C_6$-Cycloalkenyl, $C_2$-$C_8$-Alkanoyl oder $C_2$-$C_5$-Alkoxycarbonyl bedeuten; oder -CHO oder $SiR^3_4$ darstellen, worin $R^4$ $C_1$-$C_8$-Alkyl ist.

10. Titanocene gemäss Anspruch 9, dadurch gekennzeichnet, dass $R^5$, $R^6$, $R^7$ und $R^8$ unabhängig voneinander ein Wasserstoffatom, oder unsubstituiertes oder substituiertes $C_1$-$C_8$-Alkyl, $C_2$-$C_4$-Alkenyl, Benzyl, Phenyl, 2-Furyl, $C_5$- oder $C_6$-Cycloalkyl, $C_2$-$C_6$-Alkanoyl oder $C_2$-$C_5$-Alkoxycarbonyl bedeuten; oder -CHO oder $SiR^4_3$ darstellen, worin $R^4$ $C_1$-$C_4$-Alkyl ist.

11. Titanocene gemäss Anspruch 9, dadurch gekennzeichnet, dass $R^5$, $R^6$, $R^7$ und $R^8$ unabhängig voneinander ein Wasserstoffatom oder unsubstituiertes oder substituiertes $C_1$-$C_8$-Alkyl, $C_2$-$C_4$-Alkenyl, Phenyl oder 2-Furyl darstellen oder $SiR^4_3$ bedeuten, worin $R^4$ $C_1$-$C_4$-Alkyl ist.

12. Titanocene gemäss Anspruch 1, dadurch gekennzeichnet, dass in Formel IV $R^6$ oder $R^6$ und $R^7$ für H stehen.

**13.** Verfahren zur Herstellung von Titanocenen der Formel I gemäss Anspruch 1, dadurch gekennzeichnet, dass man 1 Mol einer Verbindung der Formel VI

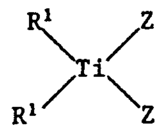

(VI),

worin $R^1$ die in Anspruch 1 angegebene Bedeutung hat und Z für Halogen, besonders Chlor, steht, mit einem Mol $LiR^2$ und einem Mol $LiR^3$ oder mit 1 Mol $Li_2QYQ$ umsetzt, wobei $R^2$, $R^3$ und QYQ die in Anspruch 1 angegebenen Bedeutungen haben, und danach die Verbindungen der Formel I in an sich bekannter Weise isoliert.

**14.** Durch Strahlung polymerisierbare Zusammensetzung, enthaltend (a) mindestens eine nichtflüchtige, monomere, oligomere oder polymere Verbindung mit mindestens einer polymerisierbaren ethylenisch ungesättigten Doppelbindung und (b) mindestens ein Titanocen der Formel I nach Anspruch 1 als Photoinitiator.

**15.** Zusammensetzung gemäss Anspruch 14, dadurch gekennzeichnet, dass zusätzlich mindestens ein von (b) verschiedener Photoinitiator (c) enthalten ist.

**16.** Zusammensetzung gemäss Anspruch 15, enthaltend als Photoinitiator (c) einen Benzoinalkylether, ein Benzophenon, ein Benzilketal, ein 4-Aroyl-1,3-dioxolan, ein Dialkoxyacetophenon, ein α-Hydroxy- oder α-Aminoacetophenon oder ein α-Hydroxycycloalkylphenylketon oder Mischungen davon als zusätzlichen Photoinitiator.

**17.** Verwendung einer Zusammensetzung gemäss Anspruch 14 zur Herstellung von Lacken, Druckfarben, Druckplatten, Resistmaterialien sowie als Bildaufzeichnungsmaterial.

**18.** Beschichtetes Substrat, das auf mindestens einer Oberfläche mit einer Zusammensetzung gemäss Anspruch 14 oder Anspruch 15 beschichtet ist.

**19.** Verfahren zur photographischen Herstellung von Reliefabbildungen, dadurch gekennzeichnet, dass man ein beschichtetes Substrat gemäss Anspruch 18 bildmässig belichtet und die unbelichteten Anteile danach mit einem Lösungsmittel entfernt.

**20.** Verwendung von Titanocenen der Formel I gemäss Anspruch 1 alleine oder zusammen mit anderen Initiatoren als Photoinitiatoren für die Photopolymerisation von nichtflüchtigen monomeren, oligomeren oder polymeren Verbindungen mit mindestens einer polymerisierbaren ethylenisch ungesättigten Doppelbindung.

**21.** Photoinitiatorengemisch, enthaltend einen Photoinitiator vom Typ der Benzoinalkylether, Benzophenone, Benzilketale, 4-Aroyl-1,3-dioxolane, Dialkoxyacetophenone, α-Hydroxyacetophenone, α-Hydroxycycloalkylphenylketone, α-Aminoacetophenone oder Mischungen hiervon und ein Titanocen der Formel I gemäss Anspruch 1.

## Claims

**1.** A titanocene of the formula I

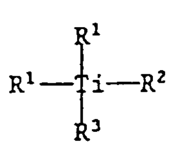

(I),

in which both $R^1$ radicals, independently of one another, are cyclopentadienyl$^{\ominus}$, indenyl$^{\ominus}$ or 4,5,6,7-

tetrahydroindenyl$^{\ominus}$, each of which is unsubstituted or mono- or polysubstituted by $C_1$-$C_{18}$alkyl or -alkoxy, $C_2$-$C_{18}$alkenyl, $C_5$-$C_8$cycloalkyl, $C_6$-$C_{16}$aryl, $C_7$-$C_{16}$aralkyl, $SiR^4_3$ , $GeR^4_3$ , cyano or halogen, or both R' radicals together are a radical of the formula II

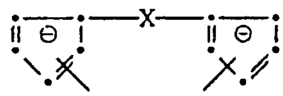

(II)

unsubstituted or substituted as above, in which X is $-(-CH_2-)-_n$ where n = 1, 2 or 3, unsubstituted or phenyl-substituted alkylidene having 2 to 12 carbon atoms, cycloalkylidene having 5 to 7 ring carbon atoms, $SiR^4_2$ , $SiR^4_2$ -O-$SiR^4_2$ , $GeR^4_2$ or $2nR^4_2$ , and $R^4$ is $C_1$-$C_{12}$alkyl, $C_5$-$C_{12}$cycloalkyl, $C_6$-$C_{16}$aryl or $C_7$-$C_{16}$aralkyl, $R^2$ is a 6-membered carbocyclic or 5 - or 6-membered heterocyclic aromatic ring which is substituted by fluorine atoms in at least one of the two ortho-positions to the titanium-carbon bond, and in which the aromatic ring may contain further substituents, $R^3$, independently, is as defined for $R^2$ or $R^2$ and $R^3$ together are a radical of the formula III

-Q-Y-Q- (III)

in which Q is a carbocyclic aromatic ring where the two bonds are each in the ortho-position to the y group and the second ortho-position to the titanium-carbon bond is in each case substituted by a fluorine atom and where Q may contain further substituents, and Y is $CH_2$, alkylidene having 2 to 12 carbon atoms, cycloalkylidene having 5 to 7 ring carbon atoms, $NR^4$, O, S, SO, $SO_2$, CO, $SiR^4_2$ , $GeR^4_2$ or $SnR^4_2$ , and $R^4$ is as defined above, wherein, in the titanocenes, $R^2$ and $R^3$ are substituted by a radical of the formula IV

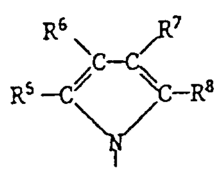

(IV)

in which $R^5$, $R^6$, $R^7$ and $R^8$, independently of one another, are a hydrogen atom, linear or branched $C_1$-$C_{18}$alkyl, $C_2$-$C_5$alkenyl, $C_7$-$C_9$aralkyl or -alkaryl, $C_8$-$C_{10}$alkaralkyl, $C_6$-$C_{10}$aryl, 2 -furyl, $C_5$-$C_8$cycloalkyl, $C_5$-$C_8$cycloalkenyl, $C_2$-$C_{12}$alkanoyl or $C_2$-$C_{12}$alkoxycarbonyl each of which is unsubstituted or substituted by $C_2$-$C_8$dialkylamino or -aminomethyl, bis-[2- ($C_1$-$C_4$alkoxy) ethyl] amino or -aminomethyl, morpholino, morpholinylmethyl, piperidino, piperidinylmethyl, N-methylpiperazino, N-methylpiperazinylmethyl, pyrrolidino, pyrrolidinylmethyl, quaternary $C_3$-$C_{10}$trialkylammonium or -ammoniummethyl, $C_1$-$C_{12}$alkoxy, $-(-OCH_2CH_2-)-_1$-O-$C_1$-$C_{16}$alkyl in which 1 is a number from 1 to 20, 1,3-dioxolan-2-yl, 4-methyl-1,3-dioxolan-2-yl, $-OCH_2CH_2O-$, $C_1$-$C_{12}$alkoxycarbonyl, $C_2$-$C_{12}$alkanoyloxy, $C_2$-$C_{12}$alkanoyl, $C_1$-$C_{12}$alkythio, halogen, cyano or $SiR^4_3$ in which $R^4$ is as defined above; or are -CHO, $-SiR^4_3$ or $-GeR^4_3$ in which $R^4$ is as defined above; or $R^5$ and $R^6$ and/or $R^7$ and $R^8$ or $R^6$ and $R^7$ in each case together are $-(CH_2)_3-$, $-(CH_2)_4-$, -CH = CH-CH = CH-, $-CH = CH-C(R^{11}) = CR-$, $-CH_2OCH_2-$or $-CH_2N(C_1$-$C_4$alkyl)$CH_2-$ in which $R^{11}$ is hydroxyl, $C_1$-$C_4$alkoxy or $C_2$-$C_4$alkanoyloxy.

**2.** A titanocene according to claim 1, wherein $R^1$ is cyclopentadienyl$^{\ominus}$ or methylcyclopentadienyl$^{\ominus}$.

**3.** A titanocene according to claim 1, wherein $R^2$ and $R^3$ are identical.

**4.** A titanocene according to claim 1, wherein the $R^2$ radical is substituted by fluorine in both the ortho-positions to the titanium-carbon bond.

**5.** A titanocene according to claim 1, wherein $R^2$ and $R^3$ are 2,6-difluorophen-1-yl to which a radical of the formula IV is bound, and which may contain a further 1 or 2 identical or different substituents.

**6.** A titanocene according to claim 5, wherein, in the formula I, both $R^1$ radicals are cyclopentadienyl$^{\ominus}$ or $C_1$-$C_4$alkyl-substituted cyclopentadienyl$^{\ominus}$, and $R^2$ and $R^3$ are radicals of the formula V

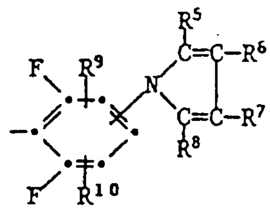

(V)

in which $R^5$, $R^6$, $R^7$ and $R^8$ are as defined in claim 1, and $R^9$ and $R^{10}$, independently of one another, are H or F.

**7.** A titanocene according to claim 6, wherein, in the formula V, the pyrrole group is bound in the ortho-position to an F atom.

**8.** A titanocene according to claim 6, wherein $R^9$ and $R^{10}$ are H.

**9.** A titanocene according to claim 1, wherein $R^5$, $R^6$, $R^7$ and $R^8$, independently of one another, are a hydrogen atom or unsubstituted or substituted $C_1$-$C_{12}$alkyl, $C_2$-$C_5$alkenyl, $C_2$- or $C_8$aralkyl or -alkaryl, phenyl, 2-furyl, $C_5$- or $C_6$cycloalkyl, $C_5$- or $C_6$cycloalkenyl, $C_2$-$C_8$alkanoyl or $C_2$-$C_5$alkoxycarbonyl; or are -CHO or $SiR^4_3$ in which $R^4$ is $C_1$-$C_8$alkyl.

**10.** A titanocene according to claim 9, wherein $R^5$, $R^6$, $R^7$ and $R^8$, independently of one another, are a hydrogen atom, or unsubstituted or substituted $C_2$-$C_8$alkyl, $C_2$-$C_4$alkenyl, benzyl, phenyl, 2-furyl, $C_5$- or $C_6$cycloalkyl, $C_2$-$C_6$alkanoyl or $C_2$-$C_5$alkoxycarbonyl; or are -CHO or $SiR^4_3$ in which $R^4$ is $C_1$-$C_4$alkyl.

**11.** A titanocene according to claim 9, wherein $R^5$, $R^6$, $R^7$ and $R^8$, independently of one another, are a hydrogen atom or unsubstituted or substituted $C_1$-$C_8$alkyl, $C_2$-$C_4$alkenyl, phenyl or 2-furyl or are $SiR^4_3$ in which $R^4$ is $C_1$-$C_4$alkyl.

**12.** A titanocene according to claim 1, wherein, in the formula IV, $R^6$ or $R^6$ and $R^7$ are H.

**13.** A process for the preparation of a titanocene of the formula I according to claim 1, wherein 1 mole of a compound of the formula VI

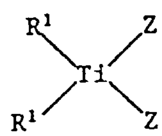

(VI),

in which $R^1$ is as defined in claim 1 and Z is halogen, particularly chlorine, is reacted with one mole of $LiR^2$ and with one mole of $LiR^3$ or with 1 mole of $Li_2QYQ$, where $R^2$, $R^3$ and QYQ are as defined in claim 1, and the compound of the formula I is then isolated in a manner known per se.

**14.** A radiation-polymerizable composition, containing (a) at least one non-volatile, monomeric, oligomeric or polymeric compound containing at least one polymerizable, ethylenically unsaturated double bond, and (b) at least one titanocene of the formula I according to claim 1 as photoinitiator.

**15.** A composition according to claim 14, wherein, in addition, at least one photoinitiator (c) other than (b) is present.

**16.** A composition according to claim 15 which contains, as photoinitiator (c), a benzoin alkyl ether, a benzophenone, a benzil ketal, a 4-aroyl-1,3-dioxolane, a dialkoxyacetophenone, an α-hydroxy- or α-aminoacetophenone or an α-hydroxycycloalkyl phenyl ketone, or a mixture thereof, as an additional photoinitiator.

**17.** The use of a composition according to claim 14 for the production of surface coatings, printing inks, printing plates, resist materials or as image-recording materials.

**18.** A coated substrate which is coated on at least one surface with a composition according to claim 14 or claim 15.

**19.** A process for photographic production of relief images, wherein a coated substrate according to claim 18 is exposed imagewise, and the unexposed areas are then removed using a solvent.

**20.** The use of a titanocene of the formula I according to claim 1, alone or together with other initiators, as photoinitiators for photopolymerization of non-volatile, monomeric, oligomeric or polymeric compounds containing at least one polymerizable, ethylenically unsaturated double bond.

**21.** A photoinitiator mixture which contains a photoinitiator of the benzoin alkyl ether, benzophenone, benzil ketal, 4-aroyl-1,3-dioxolane, dialkoxyacetophenone, α-hydroxyacetophenone, α-hydroxycycloalkyl phenyl ketone or α-aminoacetophenone type, or a mixture thereof, and a titanocene of the formula I according to claim 1.

## Revendications

**1.** Titanocènes de formule I

$$\begin{array}{c} R^1 \\ R^1 - Ti - R^2 \\ R^3 \end{array}$$

où les deux $R^1$ signifient chacun, indépendamment l'un de l'autre, un cyclopentadiényle$^{\ominus}$, un indényle$^{\ominus}$ ou un 4,5,6,7-tétrahydroindényle$^{\ominus}$ mono- ou polysubstitués par un alkyle ou un alcoxy en $C_1$-$C_{18}$, un alcényle en $C_2$-$C_{18}$, un cycloalkyle en $C_5$-$C_6$, un aryle en $C_6$-$C_{16}$, un arylalkyle en $C_7$-$C_{16}$, $SiR_3{}^4$, $GeR_3{}^4$, un cyanogène ou un halogène, ou les deux $R^1$ ensemble représentent un radical de formule II éventuellement substitué comme indiqué ci-dessus

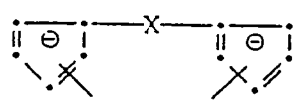

(II)

où X-$(-CH_2-)_n$ - avec n = 1, 2 ou 3, est un alkylidène comportant de 2 à 12 atomes de C éventuellement substitué par un phényle, un cycloalkylidène comportant de 5 à 7 atomes de carbone cyclique, $SiR_2{}^4$, $SiR_2{}^4$-O-$SiR_2{}^4$, $GeR_2{}^4$ ou $SnR_2{}^4$ et $R^4$ signifie un alkyle en $C_1$-$C_{12}$, un cycloalkyle en $C_5$-$C_{12}$, un aryle en $C_6$-$C_{16}$ ou un aralkyle en $C_7$-$C_{16}$,

$R^2$ signifie un cycle aromatique carbocyclique à 6 chaînons ou hétérocyclique à 5 à 6 chaînons, qui est substitué par des atomes de fluor au moins dans une des deux positions ortho par rapport à la liaison titane-carbone et le cycle aromatique pouvant comporter d'autres substituants, $R^3$ indépendamment a la signification de $R^2$ ou $R^2$ et $R^3$ ensemble sont un radical répondant à la formule III

-Q-Y-Q- (III)

où Q représente un radical aromatique carbocyclique où les deux liaisons sont chacune en position ortho par rapport au groupement Y et la deuxième position ortho par rapport à la liaison titane-carbone est substituée chaque fois par un atome de fluor, Q pouvant être porteur d'autres substituants, et Y signifie $CH_2$, un alkylidène avec 2 à 12 atomes de C, un cycloalkylidène avec 5 à 7 atomes de carbone cyclique, $NR^4$, O, S, SO, $SO_2$, CO, $SiR_2{}^4$, $GeR_2{}^4$ ou $SnR_2{}^4$ et $R^4$ a la signification indiquée ci-dessus, les titanocènes sont alors caractérisés en ce que $R^2$ et $R^3$ sont substitués par un radical de formule IV

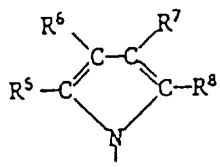

(IV)

dans laquelle $R^5$, $R^6$' $R^7$ et $R^8$ signifient chacun, indépendamment l'un de l'autre, un atome d'hydrogène, un alkyle en $C_1$-$C_{18}$, un alcényle en $C_2$-$C_5$, un aralkyle ou un alcaryle en $C_7$-$C_9$, un alcaralkyle en $C_8$-$C_{10}$, un aryle en $C_6$-$C_{10}$,le 2-furyle, un cycloalkyle en $C_5$-$C_8$, un cycloalcényle en $C_5$-$C_8$, un alcanoyle en $C_2$-$C_{12}$ ou un alcoxycarbonyle en $C_2$-$C_{12}$ linéaire ou ramifié, non substitué ou substitué par un dialkylamino-ou -aminométhyle en $C_2$-$C_8$, un bis[2-(alcoxy $C_1$-$C_4$) éthyl]amino ou -aminométhyle, un morpholino, le morpholynylméthyle, un pipéridino, le pipéridynylméthyle, un N-méthylpipérazino, le N-méthylpipérazinylméthyle, un pyrrolidino, le pyrrolidynylméthyle, un trialkylammonium ou -ammoniumméthyl quaternaire en $C_3$-$C_{10}$, un alcoxy en $C_1$-$C_{12}$, un $(-OCH_2CH_2)_\ell$-O-alkyle en $C_1$-$C_{16}$ dans lequel $\ell$ est un nombre allant de 1 à 20, le 1,3-dioxolan-2-yle, le 4-méthyl-1,3-dioxolan-2-yle, $-OCH_2CH_2O-$, un alcoxycarbonyle en $C_2$-$C_{12}$, un alcanoyloxy en $C_2$-$C_{12}$, un alcanoyle en $C_2$-$C_{12}$, un alkylthio en $C_1$-$C_{12}$, un halogène, un cyanogène ou $SiR_3{}^4$ où $R^4$ a la signification donnée auparavant; ou représentent -CHO, $-SiR_3{}^4$, ou $-GeR_3{}^4$ où $R^4$ a la signification donnée auparavant; ou $R^5$ et $R^6$ et/ou $R^7$ et $R^8$ ou $R^6$ et $R^7$ chaque fois ensemble sont $-(CH_2)_3-$, $-(CH_2)_4-$, $-CH=CH-CH=CH-$, $-CH=CH-C(R^{11})=CH-$, $-CH_2OCH_2-$ ou $-CH_2N$ (alkyl en $C_1$-$C_4$) $CH_2-$ où $R^{11}$ signifie un hydroxyle, un alcoxy en $C_1$-$C_4$, ou un alcanoyloxy en $C_2$-$C_4$.

**2.** Titanocènes selon la revendication 1, caractérisés en ce que $R^1$ est un cyclopentadiényle$^{\ominus}$ ou méthylcyclopentadiényle$^{\ominus}$.

**3.** Titanocènes selon la revendication 1, caractérisés en ce que $R^2$ et $R^3$ ont la même signification.

**4.** Titanocènes selon la revendication 1, caractérisés en ce que le radical $R^2$ est substitué par du fluor dans les deux positions ortho par rapport à liaison titane-carbone.

**5.** Titanocènes selon la revendication 1, caractérisés en ce que $R^2$ et $R^3$ correspondent au 2,6-difluorophèn-1-yle, auquel est lié un radical de formule IV et qui peut être porteur de 1 ou 2 autres substituants identiques ou différents.

**6.** Titanocènes selon la revendication 5, caractérisés en ce que les deux $R^1$ dans la formule 1 signifient le cyclopentadiényle$^{\ominus}$ ou un cyclopentadiényle$^{\ominus}$ substitué par un alkyle en C-$C_4$, et $R^2$ et $R^3$ des radicaux de formule V

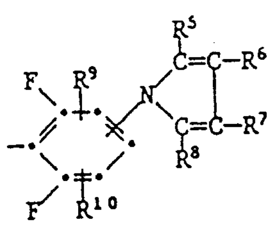

(V)

dans laquelle $R^5$, $R^6$, $R^7$ et $R^8$ ont la signification donnée dans la revendication 1, et $R^9$ et $R^{10}$ représentent chacun indépendamment l'un de l'autre H ou F.

**7.** Titanocènes selon la revendication 6, caractérisés en ce que le groupe pyrrole dans la formule V est lié en position ortho par rapport à un atome de fluor.

**8.** Titanocènes selon la revendication 6, caractérisés en ce que $R^9$ et $R^{10}$ correspondent à H.

**9.** Titanocènes selon la revendication 1, caractérisés en ce que $R^5$, $R^6$, $R^7$ et $R^8$ signifient chacun indépendamment l'un de l'autre, un atome d'hydrogène ou, substitués ou non substitués, un alkyle en

$C_1$-$C_{12}$, un alcényle en $C_2$-$C_5$, un aralkyle ou un alcaryle en $C_7$ ou en $C_8$, le phényle, le 2-furyle, un cycloalkyle en $C_5$ ou en $C_6$, un cycloalcényle en $C_5$ ou en $C_6$, un alcanoyle en $C_2$-$C_8$ ou un alcoxycarbonyle en $C_2$-$C_5$ ; ou représentent -CHO ou $SiR_3{}^4$, ou $R^4$ est un alkyle en $C_1$-$C_8$.

**10.** Titanocènes selon la revendication 9, caractérisés en ce que $R^5$, $R^6$, $R^7$ et $R^8$ signifient chacun indépendamment l'un de l'autre un atome d'hydrogène ou, substitués ou non substitués, un alkyle en $C_1$-$C_8$, un alcényle en $C_2$-$C_4$, le benzyle, le phényle, le 2-furyle, un cycloalkyle en $C_5$ ou en $C_6$, un alcanoyle en $C_2$-$C_6$ ou un alcoxycarbonyle en $C_2$-$C_5$; ou représentent -CHO ou $SiR_3{}^4$ où $R^4$ est un alkyle en $C_1$-$C_4$.

**11.** Titanocènes selon la revendication 9 caractérisés en ce que $R^5$, $R^6$, $R^7$ et $R^8$ chacun indépendamment l'un de l'autre représentent un atome d'hydrogène ou, substitués ou non substitués, un alkyle en $C_1$-$C_8$, un alcényle en $C_2$-$C_4$, un phényle -ou le 2-furyle ou signifient $SiR_3{}^4$ où $R^4$ est un alkyle en $C_1$-$C_4$.

**12.** Titanocènes selon la revendication 1 caractérisés en ce que dans la formule IV $R^6$ ou $R^6$ et $R^7$ correspondent à H.

**13.** Procédé pour la préparation de titanocènes de formule I selon la revendication 1, caractérisé en ce qu'on fait réagir une mole d'un composé de formule VI

$$\begin{matrix} R^1 & & Z \\ & Ti & \\ R^1 & & Z \end{matrix} \qquad (VI)$$

dans laquelle $R^1$ a la signification donnée dans la revendication 1 et Z représente un halogène, surtout le chlore, avec une mole de $LiR^2$ et une mole de $LiR^3$ ou avec une mole de $Li_2QYQ$, $R^2$, $R^3$ et QYQ ayant la signification donnée dans la revendication 1 et après, on isole ensuite les composés de formule I de façon connue en soi.

**14.** Composition polymérisable par irradiation contenant (a) au moins un composé non volatile monomère, oligomère ou polymère avec au moins une double liaison à insaturation éthylénique polymérisable et (b) au moins un titanocène de formule I selon la revendication 1 en tant que photoamorceur.

**15.** Composition selon la revendication 14 caractérisée en ce qu'elle renferme en plus au moins un photo-amorceur (c) différent de (b).

**16.** Composition selon la revendication 15 contenant en tant que photo-amorceur (c) un éther benzoinalkylique, une benzophénone, un benzilcétal, un 4-aroyl-1,3-dioxolanne, une dialkylacétophénone, une $\alpha$-hydroxy- ou $\alpha$-aminoacétophénone ou une $\alpha$-hydroxycycloalkulphénylcétone ou leurs mélanges en tant que photo-amorceur supplémentaire.

**17.** Utilisation d'une composition selon la revendication 14, pour la préparation de vernis, d'encres d'imprimerie, de clichés d'imprimerie, de matériaux de résists ainsi que de matériaux d'enregistrement d'images.

**18.** Substrat enduit dont au moins une surface est enduite d'une composition selon la revendication 14 ou la revendication 15.

**19.** Procédé pour la préparation Photographique de reproductions en relief caractérisé en ce qu'on irradie selon l'image un substrat enduit selon la revendication 18 et qu'on élimine ensuite les parties non exposées par un solvant.

**20.** Utilisation de titanocènes de formule I selon la revendication 1 seuls ou ensemble avec d'autres amorceurs en tant que photo-amorceurs pour la photopolymérisation de composés non volatiles monomères, oligomères ou polymères contenant au moins une double liaison polymérisable à insaturation éthylénique.

**21.** Mélange de photo-amorceurs contenant un photo-amorceur de type éther benzoinalkylique, benzophénone, benzilcétal, 4-aroyl-1,3-dioxolanne, dialkylacétophénone, α-hydroxyacétophénone, α-hydroxycycloalkylphénylcétone, α-aminocétophénone ou leurs mélanges et un titanocène de formule I selon la revendication 1.